# EUROPEAN PATENT SPECIFICATION

(11) **EP 1 948 794 B1**
(45) Date of publication and mention of the grant of the patent: **13.07.2011**
(21) Application number: 06808581.0
(22) Date of filing: 17.11.2006
(51) Int. Cl.: C12N 15/10, C12Q 1/68

(54) **A METHOD FOR IN VITRO MOLECULAR EVOLUTION OF PROTEIN FUNCTION**
VERFAHREN ZUR MOLEKULAREN EVOLUTION DER PROTEINFUNKTION IN VITRO
PROCEDE IN VITRO D'EVOLUTION MOLECULAIRE DE FONCTIONS DE PROTEINES

(30) Priority: 19.11.2005 GB 0523582
(43) Date of publication of application: 30.07.2008
(73) Proprietor: Alligator Bioscience AB, 223 70 Lund (SE)
(72) Inventor: HARALDSSON, Karin, S-227 36 Lund (SE); KARLSSON, Marie, S-222 29 Lund (SE); HAGER, Ann-Christin, Malmborg, S-252 50 Helsingborg (SE); FUREBRING, Christina, S-224 72 Lund (SE); KARLSSON, Fredrik, S-222 21 Lund (SE); ELLMARK, Peter, S-226 49 Lund (SE)
(74) Representative: Smith, Stephen Edward
(86) International application number: PCT/GB2006/004294
(87) International publication number: WO 2007/057682

(56) References cited:
- WO-A-98/32845
- WO-A2-03/097834

## Description

The present invention relates to a method for *in vitro* molecular evolution of protein function which permits control on the variability introduced into selected regions of a parent protein.

Protein function can be modified and improved *in vitro* by a variety of methods, including site directed mutagenesis (Alber et al., Nature, 5; 330(6143):41-46, 1987) combinatorial cloning (Huse et al., Science, 246:1275-1281, 1989; Marks et al., biotechnology, 10: 779-783, 1992) and random mutagenesis combined with appropriate selection systems (Barbas et al., PNAS. USA, 89: 4457-4461, 1992).

The method of random mutagenesis together with selection has been used in a number of cases to improve protein function and two different strategies exist. Firstly, randomisation of the entire gene sequence in combination with the selection of a variant (mutant) protein with desired characteristics, followed by a new round of random mutagenesis and selection. This method can then be repeated until a protein variant is found which is considered optimal (Schier R. et al., J. Mol. Biol. 1996 263 (4): 551-567). Here, the traditional route to introduce mutations is by error prone PCR (Leung et al., Technique, 1: 11-15, 1989) with a mutation rate of approximately 0.7%. Secondly, defined regions of the gene can be mutagenised with degenerate primers, which allows for mutation rates of up to 100% (Grriffiths et al., EMBO. J, 13: 3245-3260, 1994; Yang et al., J. Mol. Biol. 254: 392-403, 1995).

Random mutation has been used extensively in the field of antibody engineering. Antibody genes formed *in vivo* can be cloned *in vitro* (Larrick et al., Biochem. Biophys. Res. Commun. 160: 1250-1256, 1989) and random combinations of the genes encoding the variable heavy and light genes can be subjected to selection (Marks et al., Biotechnology, 10: 779-783, 1992). Functional antibody fragments selected by these methods can be further improved using random mutagenesis and additional rounds of selections (Schier R. et al., J. Mol. Biol. 1996 263 (4): 551-567).

Typically, the strategy of random mutagenesis is followed by selection. Variants with interesting characteristics can be selected and the mutated DNA regions from different variants, each with interesting characteristics, combined into one coding sequence (Yang et al., J. Mol. Biol. 254: 392-403, 1995).

Combinatorial pairing of genes has also been used to improve protein function, *e.g*. antibody affinity (Marks et al., Biotechnology, 10: 779-783, 1992).

Another known process for *in vitro* mutation of protein function, which is often referred to as "DNA shuffling", utilises random fragmentation of DNA and assembly of fragments into a functional coding sequence (Stemmer, Nature 370: 389-391, 1994). The DNA shuffling process generates diversity by recombination, combining useful mutations from individual genes. It has been used successfully for artificial evolution of different proteins, *e.g*. enzymes and cytokines (Chang et al. Nature Biotech. 17, 793-797, 1999; Zhang et al. Proc. Natl. Acad. Sci. USA 94, 4504-4509,1997; Christians et al. Nature Biotech. 17, 259-264, 1999). The genes are randomly fragmented using DNase I and then reassembled by recombination with each other. The starting material can be either a single gene (first randomly mutated using error-prone PCR) or naturally occurring homologous sequences (so-called family shuffling).

DNase I hydrolyses DNA preferentially at sites adjacent to pyrimidine nucleotides, therefore it is a suitable choice for random fragmentation of DNA. However, the activity is dependent on Mg or Mn ions, Mg ions restrict the fragment size to 50bp, while the Mn ions will give fragment sizes less than 50bp. Therefore, in order to have all possible sizes for recombination the gene in question needs to be treated at least twice with DNase I in the presence of either of the two different ions, followed by removal of these very same ions.

Although, in theory, it is possible to shuffle DNA between any clones, if the resulting shuffled gene is to be functional with respect to expression and activity, the clones to be shuffled have preferably to be related or even identical, with the exception of a low level of random mutations. DNA shuffling between genetically different clones will generally produce non-functional genes.

The present invention seeks to provide improved methods for *in vitro* protein evolution. In particular, the invention aims to provide a method which permits control of the degree of variability introduced in selected regions of a parent polynucleotide sequence.

Thus, according to a first aspect of the present invention, there is provided a method for generating a variant polynucleotide molecule, or population thereof, from a parent polynucleotide molecule, the method comprising the steps of
(a) providing a first population of polynucleotide molecules and a second population of polynucleotide molecules, the first and second populations together constituting plus and minus strands of a parent polynucleotide molecule;
(b) digesting the first and second populations of polynucleotide molecules with a nuclease to generate polynucleotide fragments;
(c) contacting said polynucleotide fragments generated from the plus strands with fragments generated from the minus strands (under conditions which permit annealing of fragments); and
(d) amplifying the fragments that anneal to each other to generate at least one polynucleotide molecule which differs in sequence from the parent polynucleotide molecule
wherein the degree of sequence variability in a selected region of the at least one polynucleotide molecule produced in step (d) is controlled by the addition of one or more oligonucleotides of predetermined variability, which oligonucleotides anneal to a sequence that lies between, but excludes, the 3' and 5' terminal nucleotides of the parent polynucleotide molecule.

A key advantage provided by the methods of the present invention is that they allow control of the degree of sequence variability introduced into the parent polynucleotide sequences, by the addition of one or more oligonucleotides of predetermined variability. Such oligonucleotides are able to anneal (preferably under high stringency conditions) to an internal target sequence present in one or more of the parent polynucleotide sequences.

The oligonucleotides of predetermined variability are capable of annealing to an internal sequence that lies between, but excludes, the 3' or 5' terminal nucleotide of the parent polynucleotide molecule (such that the oligonucleotides are not able to anneal to the 3' or 5'terminal nucleotides). Thus, the term 'oligonucleotides of predetermined variability' is not intended to encompass 3' or 5' end primer sequences or a full-length template. However, it will be appreciated that step (c) may additionally comprise adding primer sequences that anneal to the 3' and/or 5'ends of at least one of the parent polynucleotides under annealing conditions.

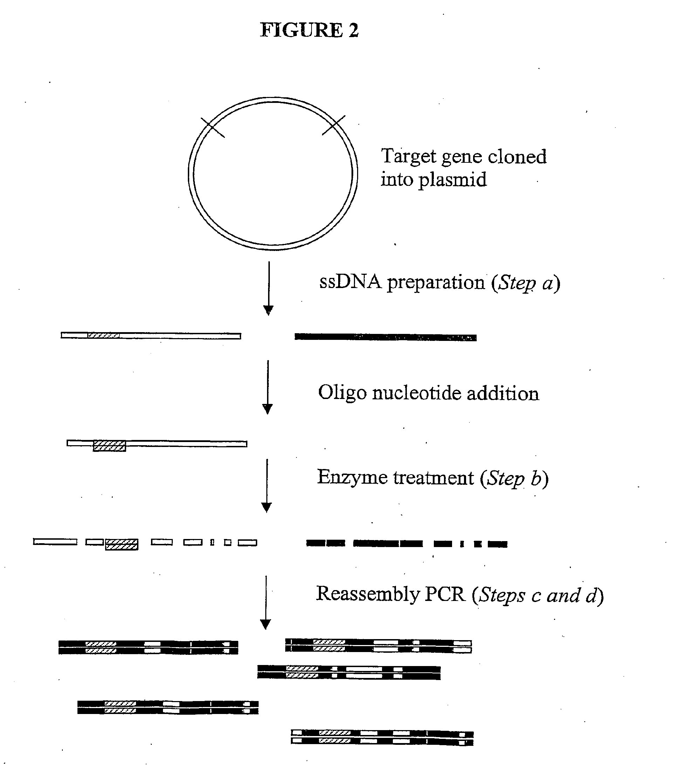

In a preferred embodiment of the method of the invention, wherein the first and second populations of polynucleotides are single-stranded, the oligonucleotides of predetermined variability are added prior to or in step (b) and the nuclease used to digest the parent polynucleotides is specific for single-stranded polynucleotides (for example, S1 nuclease, Exo I, Exo T and Mung bean nuclease). When so added, the oligonucleotides anneal/hybridise to the first and second populations of single-stranded parent polynucleotides, thereby producing double-stranded regions which are thus protected from digestion from the single-strand specific nuclease (see Figure 2). Consequently, variability within this protected sequence is controlled in the resulting variant polynucleotides produced in step (d).

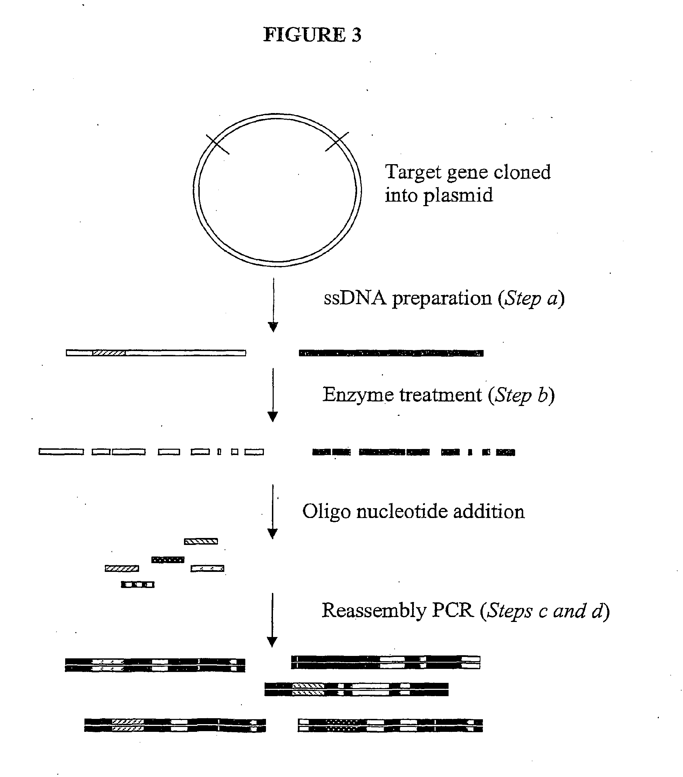

In an alternative preferred embodiment, the oligonucleotides of predetermined variability are added after step (b) and prior to or in step (c). In this embodiment, the polynucleotide fragments produced by nuclease digestion are 'spiked' with the oligonucleotides, which are then incorporated during the re-annealing/hybridisation process into the variant polynucleotides produced in step - (d) (see Figure 3). Again, it is preferred that the first and second populations of polynucleotides are single-stranded in this embodiment.

Control of the variability introduced into the variant polynucleotides produced using the method of the invention is accomplished through the use of oligonucleotides of predetermined variability. For example, oligonucleotides incorporating varying degrees of nucleotide sequence variability (from no variability to high variability) may be produced using methods well known in the art, such as error-prone PCR or using an oligonucleotide synthesiser (such as those commercially-available from MWG Biotech, Ebersberg, Germany). Thus, it will be appreciated that knowledge of the sequence of the oligonucleotides of predetermined variability is not essential; what is important is that the degree of variability within the oligonucleotides is known (at least in a relative sense, if not an absolute sense).

Advantageously, the oligonucleotides of predetermined variability share at least 90% sequence identity with the internal sequence of a parent polynucleotide sequence, for example at least 95%, 96%, 97%, 98%, 99% or 100% sequence identity. The percent sequence identity between two polynucleotides may be determined using suitable computer programs, many of which are available online (for example see www.hgmp.mrc.ac.uk/GenomeWeb/nuc-mult.html).

For example, sequence identity may be analysed using the Clustal W program (Thompson et al., (1994) Nucleic Acids Res 22, 4673-80). The parameters used may be as follows:
Fast pairwise alignment parameters: K-tuple (word) size; 1, window size; 5, gap penalty; 3, number of top diagonals; 5. Scoring method: x percent.
Multiple alignment parameters: gap open penalty; 10, gap extension penalty; 0.05. Scoring matrix: BLOSUM.

In a preferred embodiment, the oligonucleotides of predetermined variability share 100% sequence identity with the internal sequence of a parent polynucleotide sequence. Thus, the oligonucleotides may all be of a same nucleotide sequence.

In an alternative embodiment, the oligonucleotides of predetermined variability are of at least two different sequences. Preferably, the oligonucleotides are variants of the same internal sequence of a parent polynucleotide sequence.

It will be appreciated that the oligonucleotides of predetermined variability may be targeted to the same internal sequence or different internal sequences of the parent polynucleotides.

In a preferred embodiment, the oligonucleotides of predetermined variability share 100% sequence identity with, or are variants of, at least two different regions of the parent polynucleotides.

It will be appreciated that the oligonucleotides of predetermined variability may be of any length provided that they do not constitute a full-length template. Preferably, however, the oligonucleotides are between 10 and 500 nucleotides in length. More preferably, the oligonucleotides are between 50 and 200 nucleotides in length, for example about 100 nucleotides in length.

The invention provides a method for generating variant forms of a parent polynucleotide sequence.

It will be appreciated that the method of the invention may be carried out on any polynucleotide which encodes a polypeptide product, including any proteins having binding or catalytic properties, *e.g*. antibodies or parts of antibodies, enzymes or receptors. Furthermore, any polynucleotide that has a function that may be altered, such as catalytic RNA, may be mutated in accordance with the present invention. It is preferable that the parent polynucleotide encoding one or more protein motif is at least 12 nucleotides in length, more preferably at least 20 nucleotides in length, even more preferably more than 50 nucleotides in length. Polynucleotides being at least 100 nucleotides in length or even at least 200 nucleotides in length may be used. Where parent polynucleotides are used that encode large proteins such as enzymes or antibodies, these may be many hundreds or thousands of bases in length. The present invention may be carried out on any size of parent polynucleotide.

Advantageously, the altered sequence of the at least one polynucleotide molecule produced in step (d) is associated with an altered property or characteristic of the polynucleotide or polypeptide encoded thereby.

The altered property or characteristic of a polynucleotide or polypeptide generated by the method of the invention may be any variation or alteration in the normal activity of the wild type (parent) polynucleotide or of the polypeptide, protein or protein motifs it encodes. For example, the methods of the invention may be applied as follows:
(i) to modulate, either positively or negatively, the catalytic activity of an enzyme;
(ii) to modulate the binding specificity'and/or affinity of an antibody;
(iii) to modulate the binding specificity and/or affinity of a ligand-receptor interaction, *e.g*. between an interleukin and its receptor (by producing variants of the ligand and/or the receptor);
(iv) to modulate the ability of a polypeptide monomer to form multimeric formations, *e.g*. in virus coat proteins for vaccines;
(v) to modulate the ability of an immunogen to stimulate the production of specific antibodies against it; and
(vi) to modulate the stability of a protein (*e.g*. serum stability of hormones and growth factors).

Thus, it will be appreciated that the methods of the invention may be used to alter a property/function of any protein, polypeptide or polynucleotide.

Methods for testing variant polynucleotides or polypeptides generated by the method of the invention for altered properties are well known in the art. For example, selection of functional proteins from molecular libraries has been revolutionised by the development of the phage display technology (Parmley et al., Gene, 73: 305-391 1988; McCafferty et al., Nature, 348: 552-554, 1990; Barbas et al., PNAS. USA, 88: 7978-7982, 1991). In this method, the phenotype (protein) is directly linked to its corresponding genotype (DNA) and this allows for direct cloning of the genetic material, which can then be subjected to further modifications in order to improve protein function. Phage display has been used to clone functional binders from a variety of molecular libraries with up to 10¹¹ transformants in size (Griffiths et al., EMBO. J. 13: 3245-3260, 1994). Thus, phage display can be used to clone directly functional binders from molecular libraries, and can also be used to improve further the clones originally selected. Other types of viruses that have been used for surface expression of protein libraries and selections thereof are baculovirus (Boublik et al Biotechnol 13:1079-1084. 1995; Mottershead et al Biochem Biophys Res Com 238:717-722, 1997; Grabherr et al Biotechniques 22:730-735, 1997) and retrovirus (Buchholz et al Nature Biotechnol 16:951-954, 1998).

Selection of functional proteins from molecular libraries can also be performed by cell surface display. Also here, the phenotype is directly linked to its corresponding genotype. Bacterial cell surface display has been used for *e.g*. screening of improved variants of carboxymethyl cellulase (CMCase) (Kim et al Appl Environ Microbiol 66:788-93, 2000). Other cells that can be used for this purpose are yeast cells (Boder and Wittrup Nat. Biotechnol 15:553-557, 1997), COS cells (Higuchi et al J Immunol Meth 202:193-204, 1997) and insect cells (Granzerio et al J Immunol Meth 203:131-139, 1997; Ernst et al Nucleic Acids Res 26:1718-1723, 1998).

The parent polynucleotide preferably encodes one or more protein motifs. These are defined as regions or elements of polynucleotide sequence that encode a polypeptide (*i.e*. amino acid) sequence which has a characteristic protein function. For example, a protein motif may define a portion of a whole protein, such as an epitope, a cleavage site or a catalytic site *etc.*

Several searchable databases of protein motifs and potential protein motifs are available, such as MOTIF, PROSITE, SMART and BLOCKS (www.blocks.fhcrc.org).

Preferably, the selected region of the parent polynucleotide molecule in which the degree of variability is controlled corresponds to (*i.e*. encodes) one or more such protein motifs. Thus, the oligonucleotides of predetermined variability may be targeted to an internal sequence of the parent polynucleotide molecule which encodes a protein motif.

It will be appreciated by persons skilled in the art that the method of the invention may be operated using, as a parent polynucleotide, any nucleic acid starting material capable of hybridising to form double-stranded complementary nucleotide sequences, for example genomic DNA (gDNA) or complementary DNA (cDNA). Preferably, the first and second populations of polynucleotides are cDNA.

In a preferred embodiment, the first and second populations of polynucleotides are single-stranded.

Conveniently, the first population of polynucleotides consists of plus strands of parent polynucleotide molecules and second population of polynucleotides consists of minus strands of parent polynucleotide molecules. Alternatively, first and/or second population of polynucleotides may comprise both plus and minus strands of parent polynucleotide molecules.

As stated above, the method of the invention may be used to produce variant forms of any parent polynucleotide sequence.

Advantageously, the parent polynucleotide sequences are derived by mutagenesis of a single parent polynucleotide sequence, *i.e*. the parent polynucleotide sequences constitute variant forms of a single polynucleotide sequence. Random mutation of a parent polynucleotide sequence can be accomplished by any conventional method as described above, such as error-prone PCR.

In a preferred embodiment of the method of the invention, the parent polynucleotide sequences encode a ligand polypeptide. By "ligand polypeptide" we include any polypeptide which interacts either *in vivo* or *ex vivo* with another biological molecule (such as another polypeptide or a polynucleotide). Preferably, the oligonucleotides of predetermined variability share sequence identity with, or are variants of, a region of the parent polynucleotide sequences encoding an amino acid sequence which interacts, directly or indirectly, with a biological molecule, for example a binding site or modulatory site.

In a further preferred embodiment, the parent polynucleotide sequences encode an antibody or antibody fragment such as Fab-like molecules (Better et al (1988) Science 240, 1041); Fv molecules (Skerra et al (1988) Science 240, 1038); single-chain Fv (ScFv) molecules (Bird et al (1988) Science 242, 423**;** Huston et al (1988) Proc. Natl. Acad. Sci. USA 85, 5879) and single domain antibodies (dAbs) (Ward et al (1989) Nature 341, 544). In this embodiment, the oligonucleotides of predetermined variability preferably share sequence identity with, or are variants of, a region of the parent polynucleotide sequences encoding a complementarity-determining region (CDR). Alternatively, the oligonucleotides of predetermined variability may share sequence identity with, or be variants of, a region of the parent polynucleotide sequences encoding a framework polypeptide.

In a further preferred embodiment, the parent polynucleotide sequences encode an enzyme or catalytically active fragment thereof. Although the term "enzyme" is used, this is to be interpreted as also including any polypeptide having enzyme-like activity, *i.e*. a catalytic function. For example, polypeptides being part of an enzyme may still possess catalytic function. Furthermore, proteins such as interferons and cytokines are included. In this embodiment, the oligonucleotides of predetermined variability preferably share sequence identity with, or are variants of, a region of the parent polynucleotide sequences encoding the active site, or modulatory site (such as an allosteric regulatory site, *e.g*. a cofactor binding site) or a region involved in enzyme stability (such as a protease cleavage site).

In a still further preferred embodiment, the parent polynucleotide sequences encode an antigen. By "antigen", we include antigenic peptides capable of inducing an immune response when administered, either acutely or chronically, to a mammalian host. In this embodiment, the oligonucleotides of predetermined variability preferably share sequence identity with, or are variants of, a region of the parent polynucleotide sequences encoding an epitope.

It will be appreciated by persons skilled in the art that the any nuclease may be used in digestion step (b) to generate polynucleotide fragments, for example exonucleases, endonucleases or restriction enzymes, or combinations thereof. The individual digested fragments are purified, mixed and reassembled with PCR technology. The assembled (reconstituted) gene may then be cloned into an expression vector for expressing the protein. The protein may then be analysed for altered characteristics.

By 'nuclease' we mean a polypeptide, *e.g*. an enzyme or fragment thereof, having nucleolytic activity. Preferably, nuclease is an exonuclease. More preferably, the exonucleolytic activity of the polypeptide is greater than the endonucleolytic activity of the polypeptide..More preferably, the polypeptide has exonucleolytic activity but is substantially free of endonucleolytic activity.

Suitable exonucleases include BAL31, exonuclease I, exonuclease V, exonuclease VII, exonuclease T7 gene 6, bacteriophage lambda exonuclease and exonuclease Rec J_{f}.

Preferably, the first and second populations of polynucleotides are digested separately in step (b).

By controlling the parameters of the nuclease digestion reaction, the size of the polynucleotide fragments may be controlled. Determining the lengths of the polynucleotide fragments in this way avoids the necessity of having to provide a further step such as purifying the fragments of desired length from a gel.

Advantageously, at least one parameter of the reaction used for digestion of the first population of polynucleotide molecules is different from the equivalent parameter(s) used in the reaction for digestion of the second population of polynucleotide molecules. By 'equivalent parameter' we mean the same parameter used in the reaction for digestion of the other population of single-stranded polynucleotide molecules. Suitable reaction parameters which may be varied include nuclease type, nuclease concentration, reaction volume, duration of the digestion reaction, temperature of the reaction mixture, pH of the reaction mixture, length of parent polynucleotide sequences, the amount of parent polynucleotide molecules and the bluffer composition of the reaction mixture.

The use of different parameters of the reaction used for digestion of the first and second populations of polynucleotide molecules provides the advantage of increased variability in the variant polynucleotides produced by the method of the invention.

Thus, a preferred embodiment of the first aspect of the invention provides a method of combining polynucleotide fragments to generate variant polynucleotide sequences, which method comprises the steps of:
(a) digesting a (preferably linear) parent polynucleotide with a nuclease to generate a population of fragments of varying lengths;
(b) assembling a polynucleotide sequence from the sequences derived from step (a)
wherein oligonucleotides of predetermined variability are used to control the degree of variability in selected regions of the resultant polynucleotide sequences.

Preferably the method further comprises the step of (c) expressing the resulting protein encoded by the assembled polynucleotide sequence and (d) screening the protein for altered properties or characteristics.

The present invention also provides polynucleotide sequences obtained or obtainable by the method described above having an altered nucleotide sequence (preferably encoding a polypeptide having altered/desired characteristics). These polynucleotide sequences may be used for generating gene therapy vectors and replication-defective gene therapy constructs or vaccination vectors for DNA-based vaccinations. In addition, the polynucleotide sequences may be used as research tools.

The present invention also provides a polynucleotide library of sequences generated by the method described above from which a polynucleotide may be selected which encodes a protein having the altered/desired characteristics. It is preferable that the polynucleotide library is a DNA or cDNA library.

The present invention also provides proteins such as enzymes, antibodies, and receptors having characteristics different to that of the wild type produced by the method described above. These proteins may be used individually or within a pharmaceutically acceptable carrier as vaccines or medicaments for therapy, for example, as immunogens, antigens or otherwise in obtaining specific antibodies. They may also be used as research tools.

In order to obtain expression of the generated polynucleotide sequence, the polynucleotide may be incorporated in a vector having control sequences operably linked to the polynucleotide sequence to control its expression. The vectors may include other sequences such as promoters or enhancers to drive the expression of the inserted polynucleotide sequence, further polynucleotide sequences so that the protein encoded for by the polynucleotide is produced as a fusion and/or nucleic acid encoding secretion signals so that the protein produced in the host cell is secreted from the cell. The protein encoded for by the polynucleotide sequence can then be obtained by transforming the vectors into host cells in which the vector is functional, culturing the host cells so that the protein is produced and recovering the protein from the host cells or the surrounding medium. Prokaryotic and eukaryotic cells are used for this purpose in the art, including strains of *E. coli,* yeast, and eukaryotic cells such as COS or CHO cells. The choice of host cell can be used to control the properties of the protein expressed in those cells, *e.g*. controlling where the protein is deposited in the host cells or affecting properties such as its glycosylation.

The protein encoded by the polynucleotide sequence may be expressed by methods well known in the art. Conveniently, expression may be achieved by growing a host cell in culture, containing such a vector, under appropriate conditions which cause or allow expression of the protein.

Systems for cloning and expression of a protein in a variety of different host cells are well known. Suitable host cells include bacteria, eukaryotic cells such as mammalian and yeast, and baculovirus systems. Also, utilising the retrovirus system for cloning and expression is a good alternative, since this virus can be used together with a number of cell types. Mammalian cell lines available in the art for expression of a heterologous polypeptide include Chinese hamster ovary cells, HeLa cells, baby hamster kidney cells, COS cells and many others. A common, preferred bacterial host is *E. coli.*

Suitable vectors can be chosen or constructed, containing appropriate regulatory sequences, including promoter sequences, terminator fragments, polyadenylation sequences, enhancer sequences, marker genes and other sequences as appropriate. Vectors may be plasmids, viral *e.g*. phage, or phagemid, as appropriate. For further details see, for example, Molecular Cloning: a Laboratory Manual: 3rd edition, Sambrook and Russell, 2001, Cold Spring Harbor Laboratory Press. Many known techniques and protocols for manipulation of polynucleotide sequences, for example in preparation of polynucleotide constructs, mutagenesis, sequencing, introduction of DNA into cells and gene expression, and analysis of proteins, are described in detail in Current Protocols in Molecular Biology, Ausubel et al. eds., John Wiley & Sons, 1992.

The system can be used for the creation of DNA libraries comprising variable sequences which can be screened for the desired protein function in a number of ways. Enzyme function can be screened for with methods specific for the actual enzyme function *e.g*. CMCase activity, β-glucosidase activity and also thermostability. Furthermore, phage display and cell surface display may be used for screening for enzyme function (Crameri A. et al., Nature 1998 15; 391 (6664):288-291; Zhang J. H. et al., PNAS. USA 1997 94 (9): 4504-4509; Warren M.S. et al., Biochemistry 1996, 9; 35(27): 8855-8862; Kim et al., Appl Environ Microbiol 66:788-93, 2000) as well as for altered binding properties of *e.g*. antibodies (Griffith et al., EMBO J. 113: 3245-3260, 1994).

A polypeptide provided by the present invention may be used in screening for molecules which affect or modulate its activity or function. Such molecules may be useful in a therapeutic (possibly including prophylactic) context.

The present invention also provides vectors comprising polynucleotide sequences generated by the method described above.

The present inventions also provides compositions comprising either polynucleotide sequences, vectors comprising the polynucleotide sequences or polypeptides generated by the method described above and a pharmaceutically acceptable carrier or a carrier suitable for research purposes.

The present invention further provides a method comprising, following the identification of the polynucleotide or polypeptide having desired characteristics by the method described above, the manufacture of that polypeptide or polynucleotide in whole or in part, optionally in conjunction with additional polypeptides or polynucleotides.

Thus, a further aspect of the invention provides a method for making a polypeptide having altered/desired properties, the method comprising the following steps:
(a) generating variant forms of a parent polynucleotide using a method according to the first aspect of the invention;
(b) expressing the variant polynucleotides produced in step (a) to produce variant polypeptides;
(c) screening the variant polypeptides for desired properties; and
(d) selecting a polypeptide having desired properties from the variant polypeptides.

The invention further provides a polypeptide obtained by the above method.

Following the identification of a polynucleotide or polypeptide having altered/desired characteristics, these can then be manufactured to provide greater numbers by well-known techniques such as PCR, cloning and expression within a host cell.

The resulting polypeptides or polynucleotides may be used in the preparation of industrial enzymes, *e.g*. laundry detergent enzymes where an increased activity is preferred at lower temperatures. Alternatively, the manufactured polynucleotide or polypeptide may be used as a research tool, *i.e*. antibodies may be used in immunoassays, and polynucleotides may be used as hybridisation probes or primers. Alternatively, the resulting polypeptides or polynucleotides may be used in the preparation of medicaments for diagnostic use, pharmaceutical use, therapy etc. as discussed as follows.

The polypeptides or polynucleotides generated by the methods of the invention and identified as having altered characteristics can be formulated in pharmaceutical compositions. These compositions may comprise, in addition to one of the above substances, a pharmaceutically acceptable excipient, carrier, buffer, stabilizer or other materials well known to those skilled in the art. Such materials should be non-toxic and should not interfere with the efficacy of the active ingredient. The precise nature of the carrier or other material may depend on the route of administration, *e.g*. oral, intravenous, cutaneous or subcutaneous, nasal, intramuscular, intraperitoneal routes.

Pharmaceutical compositions for oral administration may be in tablet, capsule, powder or liquid form. A tablet may include a solid carrier such as gelatin or an adjuvant. Liquid pharmaceutical compositions generally include a liquid carrier such as water, petroleum, animal or vegetable oils, mineral oil or synthetic oil. Physiological saline solution, dextrose or other saccharide solution or glycols such as ethylene glycol, propylene glycol or polyethylene glycol may be included.

For intravenous, cutaneous or subcutaneous injection, or injection at the site of affliction, the active ingredient will be in the form of a parenterally acceptable aqueous solution which is pyrogen-free and has suitable pH, isotonicity and stability. Those of relevant skill in the art are well able to prepare suitable solutions using, for example, isotonic vehicles such as Sodium Chloride Injection, Ringer's Injection, Lactated Ringer's Injection. Preservatives, stabilizers, buffers, antioxidants and/or other additives may be included, as required.

Thus, the invention further provides a polynucleotide or polypeptide produced by the methods of the invention for use in medicine and the use of a polynucleotide or polypeptide produced by the methods of the invention in the preparation of a medicament for use in the treatment, therapy and/or diagnosis of a disease. Whether it is a polypeptide, *e.g*. an antibody or fragment thereof, an enzyme, a polynucleotide or nucleic acid molecule, identified following generation by the present invention that is to be given to an individual, administration is preferably in a "prophylactically effective amount" or a "therapeutically effective amount" (as the case may be, although prophylaxis may be considered therapy), this being sufficient to show benefit to the individual. The actual amount administered, and rate and time-course of administration, will depend on the nature and severity of what is being treated. Prescription of treatment, *e.g*. decisions on dosage etc, is within the responsibility of general practitioners and other medical doctors, and typically takes account of the disorder to be treated, the condition of the individual patient, the site of delivery, the method of administration and other factors known to practitioners. Examples of the techniques and protocols mentioned above can be found in Remington's Pharmaceutical Sciences, 16th edition, Osol, A. (ed), 1980.

Alternatively, targeting therapies may be used to deliver the active agent more specifically to certain types of cell, by the use of targeting systems such as antibody or cell specific ligands. Targeting may be desirable for a variety of reasons; for example if the agent is unacceptably toxic, or if it would otherwise require too high a dosage, or if it would not otherwise be able to enter the target cells.

Instead of administering these agents directly, they could be produced in the target cells by expression from an encoding gene introduced into the cells, *e.g*. in a viral vector (a variant of the VDEPT technique i.e. the activating agent, *e.g*. an enzyme, is produced in a vector by expression from encoding DNA in a viral vector). The vector could be targeted to the specific cells to be treated, or it could contain regulatory elements which are switched on more or less selectively by the target cells.

Alternatively, the agent could be administered in a precursor form, for conversion to the active form by an activating agent produced in, or targeted to, the cells to be treated. This type of approach is sometimes known as ADEPT or VDEPT; the former involving targeting the activating agent to the cells by conjugation to a cell-specific antibody, while the latter involves producing the activating agent, *e.g*. an enzyme, in a vector by expression from encoding DNA in a viral vector (see for example, EP-A-415731 and WO 90/07936).

A composition may be administered alone or in combination with other treatments, either simultaneously or sequentially dependent upon the condition to be treated.

As a further alternative, the polynucleotide identified as having desirable characteristics following generation by the method of the present invention could be used in a method of gene therapy, to treat a patient who is unable to synthesize the active polypeptide encoded by the polynucleotide or unable to synthesize it at the normal level, thereby providing the effect provided by the corresponding wild-type protein.

Vectors such as viral vectors have been used in the prior art to introduce polynucleotides into a wide variety of different target cells. Typically the vectors are exposed to the target cells so that transfection can take place in a sufficient proportion of the cells to provide a useful therapeutic or prophylactic effect from the expression of the desired polypeptide. The transfected nucleic acid may be permanently incorporated into the genome of each of the targeted tumour cells, providing long lasting effect, or alternatively the treatment may have to be repeated periodically.

A variety of vectors, both viral vectors and plasmid vectors, are known in the art, see US Patent No. 5,252,479 and WO 93/07282. In particular, a number of viruses have been used as gene transfer vectors, including papovaviruses, such as SV40, vaccinia virus, herpes viruses, including HSV and EBV, and retroviruses. Many gene therapy protocols in the prior art have used disabled murine retroviruses.

As an alternative to the use of viral vectors other known methods of introducing nucleic acid into cells includes electroporation, calcium phosphate coprecipitation, mechanical techniques such as microinjection, transfer mediated by liposomes and direct DNA uptake and receptor-mediated DNA transfer.

As mentioned above, the aim of gene therapy using nucleic acid encoding a polypeptide, or an active portion thereof, is to increase the amount of the expression product of the nucleic acid in cells in which the level of the wild-type polypeptide is absent or present only at reduced levels. Such treatment may be therapeutic in the treatment of cells which are already cancerous or prophylactic in the treatment of individuals known through screening to have a susceptibility allele and hence a predisposition to, for example, cancer.

The present invention also provides a kit for generating a polynucleotide sequence or population of sequences of desired characteristics comprising reagents for ssDNA preparation, an exonuclease and components for carrying out a PCR technique, for example, thermostable DNA (nucleotides) and a stopping device, for example, EGTA.

As outlined above the present invention conveniently provides for the creation of mutated enzyme gene sequences and their random combination to functional enzymes having desirable characteristics. As an example of this aspect of the invention, the enzyme genes are mutated by error prone PCR which results in a mutation rate of approximately 0.7%. The resulting pool of mutated enzyme genes are then digested with an exonuclease, *e.g*. BAL31, and the reaction inhibited by the addition of EGTA or by heat inactivation at different time points, resulting in a set of DNA fragments of different sizes. These may then be subjected to PCR based reassembly as described above. The resulting reassembled DNA fragments are then cloned and a gene library constructed. Clones may then be selected from this library and sequenced.

A further application of this technology is the generation of a population of variable DNA sequences which can be used for further selections and analyses. Besides encoding larger proteins, *e.g*. antibody fragments and enzymes, the DNA may encode peptides where the molecules functional characteristics can be used for the design of different selection systems. Selection of recombined DNA sequences encoding peptides has previously been described (Fisch et al., PNAS. USA 1996 Jul 23; 93 (15): 7761-7766). In addition, the variable DNA population can be used to produce a population of RNA molecules with *e.g*. catalytic activities. Vaish et al., (PNAS. USA 1998 Mar 3; 95 (5): 2158-2162) demonstrated the design of functional systems for the selection of catalytic RNA and Eckstein F (Ciba Found. Symp. 1997; 209; 207-212) has outlined the applications of catalytic RNA by the specific introduction of catalytic RNA in cells. The system may be used to further search through the sequence space in the selection of functional peptides/molecules with catalytic activities based on recombined DNA sequences.

Aspects and embodiments of the present invention will now be illustrated, by way of example, with reference to the accompanying figures. Further aspects and embodiments will be apparent to those skilled in the art.
Figure 1 shows the general principles of *in vitro* molecular evolution using the FIND™ technology of Alligator Bioscience (as described in WO 02/48351).
Figure 2 shows a preferred embodiment of the methods of the invention wherein the oligonucleotides of predetermined variability are added in step (b).
Figure 3 shows a preferred embodiment of the methods of the invention wherein the oligonucleotides of predetermined variability are added in step (c).
Figure 4 shows:
   A. Hybridisation of two different ssDNAs of different length and polarity.
   B. Digestion of the hybrid molecule with ExoI from 3'→5'.
   C. Digestion of the hybrid molecule with ExoVII from 5'→3' and 3'→5'.
Figure 5 shows a gel image of test hybridizations:
   Lane 1: 1kb DNA ladder (Invitrogen).
   Lane 2: Hybridisation of CT17 760bp and CT17 285bp in 10mM Tris.
   Lane 3: Hybridisation of CT17 760bp and CT17 285bp in 1x PCR buffer.
   Lane 4: ssDNA CT17 760bp.
   Lane 5: ssDNA CT17 285bp.
Figure 6 shows a gel image of ExoI and ExoVII digestions:
   Lane 1: Undigested CT17 760bp/ CT17 285 hybrid in ExoI buffer.
   Lane 2. CT17 760bp/CT17 285 hybrid digested with ExoI for 10 minutes.
   Lane 3. CT17 760bp/ CT17 285 hybrid digested with ExoI for 20 minutes.
   Lane 4: Undigested CT17 760bp/ CT17 285 hybrid in ExoVII buffer.
   Lane 5. CT17 760bp/ CT17 285 hybrid digested with ExoVII for 20 minutes.
   Lane 6. CT17 760bp/ CT17 285 hybrid digested with ExoVII for 30 minutes.
   Lane 7. EZload Precision Molecular Mass Standard (Bio-RAD).

### EXAMPLES

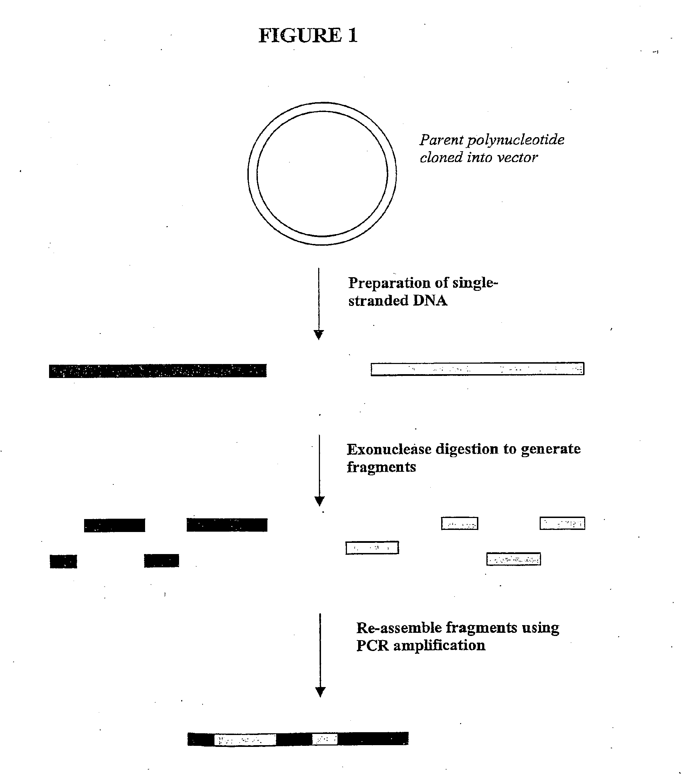

The methods of the present invention are shown schematically in Figures 1 to 3. The methods utilise the FIND™ technology of Alligator Bioscience, as described in WO 02/48351 and WO 03/097834, in the *in vitro* molecular evolution of one or more parent polynucleotide sequences.

A detailed description of exemplary embodiments of the present invention is given below.

### Example 1- The FIND™ technology

The FIND™ technology is described in WO 02/48351 and WO 03/097834.

### Reagents

AmpliTaq® polymerase was purchased from Perkin-Elmer Corp., dNTPs from Boehringer Mannheim Biochemica (Mannheim, Germany), and BAL31 Nuclease from New England Biolabs Inc. (Beverly, USA). All restriction enzymes were purchased from New England Biolabs Inc. (Beverly, USA). Ethidium bromide was purchased from Bio-Rad Laboratories (Bio-Rad Laboratories, Hercules, CA, USA). T4 DNA Ligase was purchased from New England Biolabs Inc. (Beverly, USA). EDTA and EGTA were purchased from Kebo Lab (Sweden).

All primers were designed in the laboratory and obtained from Life Technologies (Täby, Sweden) and SGS-DNA (Köping, Sweden).

### PCR

All Polymerase Chain Reactions (PCR) were carried out in an automatic thermocycler (Perkin-Elmer Cetus 480, Norwalk, CT, and USA). PCR techniques for the amplification of nucleic acid are described in US Patent No. 4,683,195. References for the general use of PCR techniques include Mullis et al., Cold Spring Harbor Symp. Quant. Biol., 51:263, (1987), Ehrlich (ed), PCR technology, Stockton Press, NY, 1989, Ehrlich et al., Science, 252:1643-1650, (1991), "PCR protocols; A Guide to Methods and Applications", Eds. Innis et al., Academic Press, New York, (1990).

### Sequencing

All constructs have been sequenced by the use of BigDye Terminator Cycle Sequencing kit (Perkin-Elmer, Elmervill, CA, USA). The sequencing was performed on an ABI Prism 377 DNA Sequencer.

### Agarose electrophoresis

Agarose electrophoresis of DNA was performed with 2% agarose gels (AGAROSE (FMC Bioproducts, Rockland, ME, USA)) with 0.25µg/ml ethidium bromide in Tris-acetate buffer (TAE-buffer 0.04M Tris-acetate, 0.001M EDTA). Samples for electrophoresis were mixed with a sterile filtrated loading buffer composed of 25% Ficoll and Bromphenolic blue and loaded into wells in a the 2% agarose gel. The electrophoresis was run at 90 V for 45 minutes unless otherwise stated in Tris-acetate buffer with 0.25 µg/ml ethidium bromide. Bands of appropriate size were gel-purified using the Qiaquick Gel Extraction Kit (Qiagen GmbH, Hilden, Germany) when needed. As molecular weight standard, DNA molecular weight marker 1 kb ladder (Gibco BRL) was used. The DNA-concentration of the gel-extracted products was estimated using a spectrophotometer.

### Bacterial Strains

The *Escherichia coli*-strain TOP10F' was used as a bacterial host for transformations. Chemically competent cells of this strain were produced basically as described Hanahan, D. 1983. Studies on transformation of Escherichia coli with plasmids. J. Mol. Biol. 166: 557-580. Electrocompetent cells of this bacterial strain were produced (Dower, W.J., J. F. Miller & C.W. Ragsdale. 1988: High efficiency transformation of E.coli by high voltage electroporation. Nucleic Acids Res. 16:6127).

### Plasmids

All genetic manipulations were performed in pFab5chis as described in Sambrook, Molecular cloning; a laboratory manual (Second Edition, Cold Spring Harbor Laboratory Press, 1989). The pFab5chis vector is designed to harbour any scFv gene inserted between SfiI and NotI sites (see Emgberg et al., 1995, Methods Mol. Biol. 51:355-376). The SfiI site is located in the pelB leader and the NotI site is located just after the VL region, such that VH-linker-VL is inserted. In this case, an antibody directed to CD40 was used.

### Primers

Two biotinylated primers surrounding the antibody gene of pFab5chis were designed with the following sequences including designated unique restriction sites:
1736 SfiI forward primer: 
5'-ATT ACT CGC GGC CCA GC▼C GGC CAT GGC CCA CAG GTC AAG
CTC GA
and 1735 NotI reversed primer:
   5'-TTA GAG CCT **GC**▼**G GCC GC**C TTG TCA TCG TCG TCC TT
(wherein '▼' designates the cleavage site)

Two non-biotinylated primers surrounding the antibody gene of pFab5chis were designed with the following sequences including designated restriction sites:
1664 SfiI forward primer: 
5'-ATT ACT CGC GGC CCA GC▼C GGC CAT GGC CCA CAG GTC AAG
CTC GA
and 1635 NotI reversed primer:
   5'- TTA GAG CCT **GC▼G GCC GC**C TTG TCA TCG TCG TCC TT

### Standard PCR

Standard PCR reactions were run at 25 cycles consisting of following profile: denaturation (94°C, 1 minute), primer annealing (55°C, 1 minute) and extension (72°C, 3 minutes). Each PCR reaction contained 10 mM Tris-HCl, pH 8.3, 50 mM KCl, 1.5 mM MgCl₂, 200 µM dNTP, 1 µM forward primer, 1 µM reverse primer, 1.25 U AmpliTaq® thermostable DNA polymerase (Perkin-Elmer Corp.), and 50 ng template in a final volume of 100 µl.

### Error Prone PCR

The error prone PCR reactions were carried out in a 10 x buffer containing 500 mM NaCl, 100 mM Tris-HCl, pH 8.8, 5mM MgCl₂ 100 µg gelatine (according to Kuipers et al., Nucleic Acids Res. 1991, Aug 25;19 (16):4558 but with MgCl₂ concentration increased from 2 mM to 5 mM).

For each 100 µl reaction the following was mixed:

| | |
|---|---|
| dATP 5 mM | 5 µl |
| dGTP 5 mM | 5 µl |
| dTTP 10 mM | 10 µl |
| dCTP 10 mM | 10 µl |
| 20 µM 3' primer | 1.5 µl |
| 20 µM 5'-primer | 1.5 µl |
| 10x Kuipers buffer | 10 µl |
| sterile mp H₂O | 46.3 µl |

The template in pFab5chis vector was added at an amount of 50 ng. 10 µl of 10 mM MnCl₂ was added and the tube was checked that no precipitation of MnO₂ occurred. At last 5 Units of Taq enzyme was added. The error prone PCR was run at the following temperatures for 25 cycles without a hot start: 94°C 1', 45 °C 1', 72 °C 1', + 72 °C for 7 minutes. The resulting product was an error proned (*i.e*. mutated) insert of 750 bp. This insert was purified with Gibco PCR purification kit, before further treatment.

### Generation of single-stranded DNA by biotinylated primers

The fragment of interest was amplified by two separate PCR reactions. These reactions can be standard PCR as described above or error prone PCR also as described above. The primers should be designed so that in one reaction the forward primer is biotinylated and in the other reaction the reverse primer is biotinylated. For example, PCR reactions with A) primers 1736 and 1635 and B) primers 1664 and 1735, with the above mentioned profile was performed for 25 cycles with pFab5chis-antibody as template. This yielded PCR-products of approximately 750 bp: in A the upper strand was biotinylated; and in B the lower strand was biotinylated.

The non-biotinylated strands were retrieved by purification using a solid matrix coated with streptavidin *e.g*. Dynabeads. The magnetic beads are washed and equilibrated with PBS/1% BSA and B&W buffer containing 5 mM Tris pH 7.5, 1 M NaCl, and 0.5 mM EGTA. 100 µl of each PCR product is mixed with 100 µl beads dissolved in 2 x B&W buffer and incubated at room temperature for 15 minutes with rotation. Unbound PCR products are removed by careful washing twice with B&W. The non-biotinylated strand of the captured DNA is eluted by alkaline denaturation by letting the DNA incubate with 25 µl 0.1 M NaOH for 10 minutes in room temperature. The solution is separated from the beads and neutralised with 7.5 µl 0.33 M HCl and 2.5 µl 1 M Tris pH 8.

### Generation of single-stranded DNA using phage

The fragment of interest was cloned into bacteriophage M13 vectors M13mp18 and M13mp19 using PstI/HindIII restriction enzymes. The bacteriophage were propagated using *Escherichia coli*-strain TOP10F' according to conventional methods. Single-stranded DNA for the upper strand was prepared from bacteriophage vector M13mp18 and single-stranded DNA for the lower strand was prepared from bacteriophage vector M13mp19. Briefly, 1.5 ml of an infected bacterial culture was centrifuged at 12 000g for 5 minutes at 4°C. The supernatant was precipitated with 200 µl 20% PEG8000/2.5 M NaCl. The pelleted bacteriophage was resuspended in 100 µl TE. 50 µl phenol equilibrated with Tris-Cl (pH 8.0) was added and the sample was vortexed. After centrifugation at 12 000g for 1 minute at RT the upper phase, containing the DNA, was transferred and precipitated with ethanol. The DNA pellet was dissolved in 50 µl TE (pH 8.0) and stored at -20°C. (Sambrook et al. Molecular Cloning, A laboratory manual 2nd edition. Cold Spring Habor Laboratory Press. 1989, chapter 4). Single-stranded DNA prepared from phage is circular and must be opened prior to BAL31 treatment. This can be performed with an endonuclease able to cleave single-stranded DNA.

### Generation of single-stranded DNA using asymmetric PCR

PCR products are purified using a spin column to remove excess primers from the previous PCR. 150 ng of the purified product is used as template in a linear amplification carried out in 100 µl of 1xGeneAmp® 10x PCR buffer containing 1.5 mM MgCl2 (Applied Biosystems), 200 µM of each dNTP (New England BioLabs), 1.25 U AmpliTaq® DNA Polymerase (Applied Biosystems) and 1.0 µM of a single primer. PCR cycle conditions are: denaturation at 94°C for 1 minute, 35 cycles of 94°C for 30 seconds, 55°C for 30 seconds, 72°C for 1 minute followed by extension at 72°C for 7 minutes.

Asymmetric PCR products are size separated from double stranded template on a 1 % agarose gel and purified using Qiaquick Gel Extraction Kit (Qiagen).

### Generation of single-stranded DNA using Lambda exonuclease.

Initially a dsDNA fragment is produced using standard PCR reactions creating a DNA with unique restriction enzyme (RE) sites in the 5' and 3'-end respectively. The PCR reaction is divided in two and RE digested respectively to create a 5' phosphorylation preferentially with restriction enzymes creating 3' overhang or blunt ends. The Digestion is performed in suitable buffer and over night to accomplish complete digestion. If an enzyme creating a 5' overhang has to be used the overhang can be filled in using a DNA polymerase. After purification 1-4 µg dsDNA is treated with 10U of Lambda exonuclease (eg Strandase™ from Novagen or Lambda exonuclease from NEB) in accompanied specific buffer for 30 min at 37°C and the reaction is stopped at 75°C for 10 min. The ssDNA is further separated from any dsDNA residues on an agarose gel using standard gel extraction methods.

### Generation of single-stranded fragmented DNA using BAL 31

The ssDNA strands (containing upper and lower strands, respectively) were subjected to separate enzymatic treatment using *e.g.* BAL 31 (*i.e*. upper strands were digested separately from lower strands). Each digestion reaction contained 0.02 µg/µl ssDNA, 600 mM NaCl, 20 mM Tris-HCl, 12 mM CaCl₂, 12 mM MgCl₂, 1 mM EDTA pH 8.0 and BAL 31 at various enzyme concentrations ranging from 0.1 - 5 U/ml. The reactions were incubated at 30°C and fractions of digested ssDNA were collected sequentially at 10, 30, 60 and 120 seconds or longer. The reactions were stopped by addition of EDTA and heat treatment at 65°C for 10 minutes. The ssDNA fragments were purified by phenol/chloroform extraction and ethanol precipitated. The ssDNA are resuspended in 10 mM Tris pH 8.0.

The digestion pattern was evaluated by 1 % agarose gel electrophoresis.

### Purification of digestion produced fragments:

Digested DNA fragments were purified by phenol/chloroform/isoamylalcohol extraction. 50 µl of buffered phenol was added to each tube of 100 µl sample together with 50 µl of a mixture of chloroform and isoamylalcohol (24:1). The tubes were vortexed for 30 seconds and then centrifuged for 1 minute in a microfuge at 14000 r.p.m. The upper phase was then collected and mixed with 2.5 volumes of 99.5% Ethanol (1/10 was 3M Sodium Acetate, pH 5.2). The DNA was precipitated for 1 hour in -80 °C. The DNA was then pelleted by centrifugation for 30 minutes in a microfuge at 14.000 r.p.m. The pellet was washed once with 70% ethanol and then re-dissolved in 10 µl of sterile water.

### Analysis of digestion produced purified fragments on agarose gel

5 µl of the dissolved pellet from each time point and from the blank were mixed with 2.5 µl of loading buffer (25% Ficoll and Bromphenolic blue) and loaded into wells in a 2% agarose gel. The electrophoresis of the different time points was performed as above.

### Reassembly of full length fragments

Reassembly of the ssDNA fragments is achieved by two sequential PCR reactions. The first PCR reaction should contain 10 mM Tris-HCl, pH 8.3, 50 mM KCl, 1.5 mM MgCl₂, 200 µM dNTP, 0.3 U Taq polymerase and 2 µl BAL31 treated sample, all in a final volume of 25 µl, and subjected to 5 cycles with the following profile: 94 °C for 1 minute, 50 °C for 1 minute and 72 °C for 2 minutes + 72 °C for 5 minutes. The second PCR reaction should contain 10 mM Tris-HCl, pH 8.3, 50 mM KCl, 1.5 mM MgCl₂, 200 µM dNTP, 0.6 U Taq polymerase, 1 µM forward primer, 1 µM reverse primer, and 5 µl sample from the first PCR reaction, all in a final volume of 50 µl, and subjected to 15 cycles with the following profile: 94 °C for 1 minute, 55 °C for 1 minute and 72 °C for 2 minutes + 72 °C for 7 minutes.

The resulting products can be evaluated by agarose gel electrophoresis.

### Restriction digestion of reassembled fragment and plasmid with SfiI and NotI

The reassembled fragment and the plasmid pFab5chis were first cleaved with SfiI by using NEB buffer 2 including BSA and 11 U enzyme/µg DNA. The reaction was carried out for 4 h at 50°C. After this the DNA was cleaved with NotI by adding conversion buffer and 6 U enzyme/µg DNA. This reaction was carried out for 37°C overnight.

### Gel purification of restriction digested vector and restriction digested reassembled fragment

The cleavage reactions were analysed on a 1% agarose gel. The restriction digested insert showed a cleavage product of about 750 bp. This corresponds well with the expected size. The band of the cleaved insert and plasmid was cut out and gel-extracted as previously described.

### Ligation of reassembled restriction digested fragment with restriction digested pFab5chis

Purified cleaved pFab5chis was ligated with purified reassembled restriction digested fragment at 12°C water bath for 16 hours. 50 µl of the vector was mixed with 50 µl of the insert and 15 µl of 10x buffer (supplied with the enzyme), 7.5 µl ligase (5 U/µl) and sterile water to a final volume of 150µl. A ligation of restriction digested pFab5chis without any insert was also performed in the same manner.

### Transformation of chemically competent E coli TOP10F' with the ligated reassembled insert and pFab5chis

The ligation reactions were purified by phenol/chloroform extraction as described above. The upper phase from the extraction was collected and mixed with 2.5 volumes of 99.5% Ethanol (1/10 was 3M Sodium Acetate, pH 5.2). The DNA was precipitated for 1 hour in -80 °C. The DNA was then pelleted by centrifugation for 30 minutes in a microfuge at 14.000 r.p.m. The pellet was washed once with 70% ethanol and then re-dissolved in 10 µl of sterile water. 5 µl of each ligation was separately mixed with 95 µl chemically competent *E coli* TOP10F' incubated on ice for 1 hour and then transformed (Sambrook et al. Molecular Cloning, A laboratory manual 2nd edition. Cold Spring Habor Laboratory Press, 1989). After one hour's growth the bacteria from the two transformations were spread onto ampicillin containing agar plates (100 µg/ml). The plates were grown upside-down in a 37°C incubator for 14 hours.

### Example 2 - Control of variability using oligonucleotides of predetermined variability I

### Introduction

The rationale for this set of experiments were that in many cases there would be an interest in either specifically mutating an area of interest as in CDR regions of an antibody and keeping the framework unchanged or adding non-mutated regions such as an active site of an enzyme inhibiting recombination events in this area.

The test genes used here were A2.30 and A2.54 (Ellmark et al. 2002 Molecular Immunology 39:349-356), two scFv clones with specificity for CD40.

### Materials and Methods

### Production of oligonucleotides of predetermined variability

Oligonucleotides of predetermined variability, corresponding to mutated forms of CDR2 of A2.30, were produced as follows.

Two sequential rounds of PCR with ErrorProne conditions (see Table 1a and 1b) was performed on the A2.30 clone using primers #137 and #138 (see Table 1c) creating mutated PCR products covering bp 56 to bp 352 of the A2.30. A third PCR with ErrorProne conditions was performed using primers #351 and #357* or #354* and #350 (* indicating a 5'-biotin labelling) creating CDR2 fragments covering the internal sequence from bp 104 to bp 221 of the A2.30 clone. These PCR products were further mutated using the Gene Morph™ PCR Mutagenesis Kit (Stratagene) using the same primers. From the biotinylated PCR products ssDNA was purified using the µMACS Strepatavidin kit (Miltenyi Biotec) and further purification was made on agarose gels where ssDNA was recovered using Recochips (TaKaRa). The ssDNA was precipitated with NaAc/ethanol and then redissolved in 10mM Tris-HCl pH 8.0.

**Table 1(a)**

| *Reagent (producer)* | *Final concentration* |
|---|---|
| Geneamp 10xPCR Gold Buffer (Applied Biosystems) | 1x |
| 25 mM MgCl1₂ (Applied Biosystems) | 7mM |
| 0.1% (w/v) gelatine (Sigma) | 0.01% |
| 5mM dATP (New England Biolabs) | 0.2mM |
| 5mM dGTP (New England Biolabs) | 0.2mM |
| 10mM dTTP (New England Biolabs) | 1mM |
| 10mM dCTP (New England Biolabs) | 1mM |
| 20uM oligonucleotide primer | 300nM |
| 20uM oligonucleotide primer | 300nM |
| 5mM MnCl₂ (Merck) | 0.5mM |
| 5U/uL AmpliTaq Gold (Applied Biosystems) | 0.025U/uL |
| DNA | 4 ng or 4uL of PCR product |

**Table 1(b)**

| *Cycles* | *Temperature* | *Time* |
|---|---|---|
| 1 | 94 °C | 7 min |
| | 94 °C | 30 sec |
| 30 | 45 °C | 30 sec |
| | 72 °C | 30 sec |
| 1 | 72 °C | 7 min |

**Table 1(c)**

| *Primer#* | *Sequence* |
|---|---|
| 137 | CGC GAA TTG GCC CAG CCG GCC ATG GCC GAG GTG CAG CTG TTG GAG |
| 138 | AGA TGG GGG ACT AGT GCT GCT CAC GGT GAC |
| 350 | CCTGGAGCCTGGCGGACCCA |
| 351 | GCTGGGTCCGCCAGGCTCCA |
| 354* | BIO-GACTCTCCTGTGCAGCCTCT |
| 357* | BIO-TTGTCTCTGGAGATGGTGAA |
| 226 | CTCACTATAGGGCGAATTGG |
| 415 | TTCAGATCTCGAGGTGCAGCTGTTGGAG |
| 224 | CCTATTGCCTACGGCAGCC |
| 332* | BIO-CCTATTGCCTACGGCAGCC |
| 333* | BIO-CTCACTATAGGGCGAATTGG |

### Production of single-stranded parent polynucleotides (Step 'a')

A standard PCR reaction (table 2a and 2b) was made on A2.30 and A2.54 clones with primers #224 and #333* or #332* and #226 (* indicating a 5'-biotin labelling). ssDNA, which served as the parent polynucleotides, was purified as described above.

### Digestion of single-stranded parent polynucleotides (Step 'b')

Exonuclease treatments were performed on the separate sense and anti-sense strands as shown in table 3 in buffer systems indicated by producer.

**Table 2a**

| *Reagent (producer)* | *Final concentration* |
|---|---|
| Geneamp 10xPCR Gold Buffer (Applied Biosystems) | 1x |
| 25 mM MgCl₂ (Applied Biosystems) | 3mM |
| 10mM dNTP (New England Biolabs) | 0.2mM |
| 20uM oligonucleotide primer | 500nM |
| 20uM oligonucleotide primer | 500nM |
| 5U/uL AmpliTaq Gold (Applied Biosystems) | 0.025U/uL |
| DNA | 4ng |

**Table 2(b)**

| *Cycles* | *Temperature* | *Time* |
|---|---|---|
| 1 | 94 °C | 7 min |
| | 94 °C | 30 sec |
| 30 | 58 °C | 30 sec |
| | 72 °C | 60 see |
| 1 | 72 °C | 7 min |

**Table 3**

| *Exonuclease (producer)* | *Amount enzyme*/*µg ssDNA* | *Time* |
|---|---|---|
| ExoI (New England Biolabs) | 100 U/µg | 10 min |
| ExoV (USB) | 25 U/µg | 30 min |
| ExoVII (USB) | 5 U/µg | 30 min |

### Generation of variant polynucleotides (Steps 'c' and 'd')

Reassembly was achieved in two stages. In the first reassembly reaction (PCR1, table 4a and 4b) 7.5ng exonuclease fragmented sense and anti-sense ssDNA from A2-30 and A2-54, respectively, was mixed with 5ng CDR2 sense fragments (the latter constituting oligonucleotides of predetermined variability; see above). After 25 cycles of PCR1 the entire reaction mixture was added to a second PCR reaction for amplification, wherein primers were added to enable the formation of full-length polynucleotides (PCR2 table 4a and 4b).

The resulting PCR products were ligated in a pGEM-T Vector System (Promega) and sequenced.

**Table 4(a)**

| *Reagent (producer)* | *Final concentration PCR1* | *Final concentration PCR2* |
|---|---|---|
| Geneamp 10xPCR Gold Buffer (Applied Biosystems) | 1x | 1x |
| 25 mM MgCl₂ (Applied Biosystems) | 1.5mM | 1.5mM |
| 1.25mM dNTP (New England Biolabs) | 0.2mM | 0.2mM |
| 20uM oligonucleotide primer #415 | - | 1mM |
| 20uM oligonucleotide primer #226 | - | 1mM |
| 5U/uL AmpliTaq Gold (Applied Biosystems) | 0.01215U/ul | 0.025U/uL |
| DNA | 35ng (see text) | all of PCR1 |

**Table 4(b)**

| *PCR1* | | | *PCR2* | | |
|---|---|---|---|---|---|
| *Cycles* | *Temperature* | *Time* | *Cycles* | *Temperature* | *Time* |
| 1 | 95 °C | 7 min | 1 | 95 °C | 7 min |
| | 94 °C | 30 sec | | 94 °C | 30 sec |
| 25 | 50 °C | 45 sec | 30 | 58 °C | 45 sec |
| | 72 °C | 60 sec | | 72 °C | 120 sec |
| 1 | 72 °C | 7 min | 1 | 72 °C | 7 min |

### Results and conclusions

Twenty clones produced as described above, using the method of the invention, were sequenced and analysed for mutations compared to both A.2-30 and A.2-54.

The overall mutation frequency was 1 mutation/1000bp, which corresponds to a normal frequency of mutation with standard PCR amplification (*i.e*. not error prone PCR).

However, seven out of the twenty clones (35%) had one or two mutations in the internal 78 bp CDR2 region. This is clearly above the probability for PCR-induced mutations alone and can therefore only be explained as induced by the addition in step (c) of the pre-mutated CDR2 oligonucleotides (*i.e.* the oligonucleotides of predetermined variability). All of the clones with mutations in the CDR2 region showed recombinations between the two initial clones A2.30 and A2.54. The number of recombinations in these clones ranged from one to four. The overall recombination frequency of the library was 1.4 recombinations per sequence.

In conclusion, this experiment demonstrates that oligonucleotides of predetermined variability may be used to selectively increase variability within a selected region (CDR2) of a parent polynucleotide encoding an scFv molecule.

### Example 3 - Control of variability using oligonucleotides of predetermined variability II

### Introduction

The following experiment was also performed using the A2.30 and A2.54 scFv clones.

### Materials and methods

### Production of oligonucleotides of predetermined variability

Oligonucleotides of predetermined variability, corresponding to mutated CDR1, CDR2, CDR3 and CDR1+2 ssDNA fragments, were produced as follows.

Two sequential rounds of PCR-with ErrorProne conditions (table 5a and 5b) was performed on the A2.30 clone using primers #137 and #138 (see table 5c) creating mutated PCR products covering bp 56 to bp 352 of the A2.30. A third PCR with ErrorProne conditions was performed using primers creating the fragments shown in table 6. These PCR products were further mutated using the Gene Morph™ PCR Mutagenesis Kit (Stratagene) using the same primers as above and indicated in table 6.

After ligation in a pGEM-T Vector System (Promega) and sequencing, the mutation frequency compared to A2-30 was calculated as shown in table 7.

**Table 5(a)**

| *Reagent (producer)* | *Final concentration* |
|---|---|
| Geneamp 10xPCR Gold Buffer (Applied Biosystems) | 1x |
| 25 mM MgCl₂ (Applied Biosystems) | 7mM |
| 0.1% (w/v) gelatine (Sigma) | 0.01% |
| 5mM dATP (New England Biolabs) | 0.2mM |
| 5mM dGTP (New England Biolabs) | 0.2mM |
| 10mM dTTP (New England Biolabs) | 1mM |
| 10mM dCTP (New England Biolabs) | 1mM |
| 20uM oligonucleotide primer | 300nM |
| 20uM oligonucleotide primer | 300nM |
| 5mM MnCl₂ (Merck) | 0.5mM |
| 5U/uL AmpliTaq Gold (Applied Biosystems) | 0.025U/uL |
| DNA | 4 ng or 4µL of PCR product |

**Table 5(b)**

| *Cycles* | *Temperature* | *Time* |
|---|---|---|
| 1 | 94 °C | 7 min |
| | 94 °C | 30 sec |
| 30 | 45 °C | 30 sec |
| | 72 °C | 30 sec |
| 1 | 72 °C | 7 min |

**Table 5(c)**

| *Primer #* | *Sequence* |
|---|---|
| 137 | CGC GAA TTG GCC CAG CCG GCC ATG GCC GAG GTG CAG CTG TTG GAG |
| 138 | AGA TGG GGG ACT AGT GCT GCT CAC GGT GAC |
| 349 | GACTCTCCTGTGCAGCCTCT |
| 350 | CCTGGAGCCTGGCGGACCCA |
| 351 | GCTGGGTCCGCCAGGCTCCA |
| 352 | TTGTCTCTGGAGATGGTGAA |
| 354* | BIO-GACTCTCCTGTGCAGCCTCT |
| 355* | BIO-CCTGGAGCCTGGCGGACCCA |
| 356* | BIO-GCTGGGTCCGCCAGGCTCCA |
| 357* | BIO-TTGTCTCTGGAGATGGTGAA |
| 226 | CTCACTATAGGGCGAATTGG |
| 415 | TTCAGATCTCGAGGTGCAGCTGTTGGAG |
| 224 | CCTATTGCCTACGGCAGCC |
| 332* | BIO-CCTATTGCCTACGGCAGCC |
| 333* | BIO-CTCACTATAGGGCGAATTGG |
| 384 | CACTGCCGTGTATTACTGT |
| 386* | BIO-CAGTGTACCTTGGCCCCA |

**Table 6**

| *Mutated fragment* | *Position in model gene including primers (bp)* | *Primer # used to purify sense strands* | | *Primer # used to purify anti-sense strands* | |
|---|---|---|---|---|---|
| CDR1 | 56-125 | 349 | 355* | 354* | 350 |
| CDR2 | 104-221 | 351 | 357* | 356* | 352 |
| CDR3 | 270-352 | 384 | 386* | | |

| | | | | | |
|---|---|---|---|---|---|
| *indicating 5'-biotin labelling of the oligonucleotide | | | | | |

**Table 7**

| *Fragment* | *Mutation frequency* |
|---|---|
| CDR1 | 2.7/100 bp |
| CDR2 | 0.7/100 bp |
| CDR3 | |

From the biotinylated PCR products indicated in table 6, ssDNA was purified using the µMACS Strepatavidin kit (Miltenyi Biotec). Further purification was carried out on agarose gels, from which ssDNA was recovered using Recochips (TaKaRa).

The ssDNA, which served as the oligonucleotides of predetermined variability in the following experiment, was precipitated with NaAc/ethanol and then redissolved in 10mM Tris-HCl pH 8.0.

### Production of parent polynucleotides (Step 'a')

A standard PCR reaction (table 8) was made on A2.30 and A2.54 with indicated primers (table 9). ssDNA, which served as the parent polynucleotides, was purified as described above.

### Digestion of single-stranded parent polynucleotides (Step 'b')

Exonuclease treatments were performed on the separate sense and anti-sense strands as shown in table 10.

**Table 8**

| *Reagent (producer)* | *Final concentration* |
|---|---|
| Geneamp 10xPCR Gold Buffer (Applied Biosystems) | 1x |
| 25 mM MgCl₂ (Applied Biosystems) | 3mM |
| 10mM dNTP (New England Biolabs) | 0.2mM |
| 20uM oligonucleotide primer | 500nM |
| 20uM oligonucleotide primer | 500nM |
| 5U/uL AmpliTaq Gold (Applied Biosystems) | 0.025U/uL |
| DNA | 4ng |

**Table 9**

| *DNA* | *Primers used to purify sense strands* | | *Primers used to purify anti-sense strands* | |
|---|---|---|---|---|
| A2.30 | 224 | 333* | 332* | 226 |
| A2.54 | 224 | 333* | 332* | 226 |

| | | | | |
|---|---|---|---|---|
| * indicating 5'-biotin labelling of the oligonucleotide | | | | |

**Table 10**

| *Exonuclease* | *Amount enzyme*/*µg ssDNA* | *Time* |
|---|---|---|
| ExoI | 100 U/µg | 10 min |
| ExoV | 25 U/µg | 30 min |
| ExoVII | 5 U/µg | 30 min |

### Generation of variant polynucleotides (Steps 'c' and 'd')

A set of libraries was made. Reassembly PCRs were made in two stages. In the first, PCR1, exonuclease fragmented sense and anti-sense ssDNA from A2-30 and A2-54, respectively, was mixed with oligonucleotides corresponding to mutated forms of CDR1, CDR2 and/or CDR3 ('oligonucleotides of predetermined variability'), as indicated in table 11a and 13b.

After 25 cycles of PCR1 (table 12a and 12b) the entire reaction mixture was added to a second reaction (PCR2; table 12a and 12b), which also contained the end specific primers, and run for 20 cycles. The PCR products were ligated in a pGEM-T Vector System (Promega) and sequenced.

**Table 11(a)**

| *Library* | *ng exonuclease treated A2.30 sense*/*anti-sense* | *ng exonuclease ng mutated treated A2.54 sense*/*anti-sense* | *CDR1 fragment sense*/*anti-sense* | *ng mutated CDR2 fragment sense*/*anti-sense* |
|---|---|---|---|---|
| A | 7.5/7.5 | 7.5/7.5 | 0.75/0.75 | - |
| B | 7.5/7.5 | 7.5/7.5 | - | 1.25/1.25 |
| C | 7.5/7.5 | 7.5/7.5 | - | 1.25/- |
| D | 7.5/7.5 | 7.5/7.5 | - | 2.5/- |
| E | 7.5/7.5 | 7.5/7.5 | - | 5/- |

**Table 11(b)**

| *Library* | *ng exonuclease treated A2.30 sense*/*anti-sense* | *ng exonuclease treated A2.54 sense*/*anti-sense* | *ng mutated CDR1*/*CDR2*/*CDR3 sense fragments* |
|---|---|---|---|
| F | 30/30 | 30/30 | 3/5/3.6 |
| G | 30/30 | 30/30 | 3/5/3.6 |

**Table 12(a)**

| *Reagent (producer)* | *Final concentration PCR1* | *Final concentration PCR2* |
|---|---|---|
| Geneamp 10xPCR Gold Buffer (Applied Biosystems) | 1x | 1x |
| 25 mM MgCl₂ (Applied Biosystems) | 1.5mM | 1.5mM |
| 1.25mM dNTP (New England Biolabs) | 0.2mM | 0.2mM |
| 20uM oligonucleotide primer #415 | - | 1mM |
| 20uM oligonucleotide primer #226 | - | 1mM |
| 5U/uL AmpliTaq Gold (Applied Biosystems) | 0.01215U/ul | 0.025U/uL |
| DNA | see table 13a and 13b | all of PCR1 |

**Table 12(b)**

| *PCR1* | | | *PCR2* | | |
|---|---|---|---|---|---|
| *Cycles* | *Temperature* | *Time* | *Cycles* | *Temperature* | *Time* |
| 1 | 95 °C | 7 min | 1 | 95 °C | 7 min |
| | 94 °C | 30 sec | | 94 °C | 30 sec |
| 25 | 50 °C | 45 sec | 30 | 58 °C | 45 sec |
| | 72 °C | 60 sec | | 72 °C | 120 sec |
| 1 | 72 °C | 7 min | 1 | 72 °C | 7 min |

### Results and conclusions

The overall mutation frequency was 1 mutation/1000bp, which corresponds to a normal frequency of mutation with standard PCR amplification (*i.e*. not error prone PCR).

Between 11% and 56% of the clones in the different libraries showed mutations in their CDR regions corresponding to the added oligonucleotides of predetermined variability (table 13). The mutated stretches were 30 bp, 78 bp and 46 bp for CDR1, CDR2 and CDR3, respectively. The incidence of mutation in these areas after addition of the CDR fragments was clearly above the probability of PCR-induced mutations alone and can therefore only be explained by the addition of the pre-mutated oligonucleotides.

**Table 13**

| *Library (clones analysed)* | *Sequences mutated in the CDR1*/*CDR2*/ *CDR3* | *Clones with CDR mutation regions mutations*/*1000bp* | *Mutation frequency in gene (-CDR region(s))* | *Overall recombinations* / *sequence* |
|---|---|---|---|---|
| A (19) | 2/na/na | 11 % | 0.71 | 1.9 |
| B (18) | na/6/na | 33% | 0.86 | 2.4 |
| C (16) | na/3/na | 19% | 0.90 | 2.6 |
| D (18) | na/2/na | 11 % | 0.84 | 2.2 |
| E (20) | na/7/na | 35% | 1.13 | 1.3 |
| F(16) | 4/2/4^ | 56% | 0.79 | 2.6 |
| G(20) | 2/3/3" | 35% | 0.85 | 2.1 |

| | | | | |
|---|---|---|---|---|
| na = not applicable ^ one clone with both CDR2 and CDR3 mutations " one clone with both CDR1 and CDR3 mutations | | | | |

In conclusion, this experiment demonstrates that oligonucleotides of predetermined variability may be used to selectively increase variability within multiple selected regions (CDR1, CDR2 and CDR3) of a parent polynucleotide encoding an scFv molecule.

### Example 4 - Control of variability using oligonucleotides of predetermined variability III

### Introduction

The following experiments were performed to demonstrate the protection of a region/regions of a nucleotide sequence from degradation with exonucleases. The rationale was to be able to protect regions in a gene from recombination in a FIND™ reaction by keeping the original sequence in these regions undigested by exonucleases. In the following FIND™ reaction these undigested regions are always of parental type and thus unrecombined.

### Experimental layout

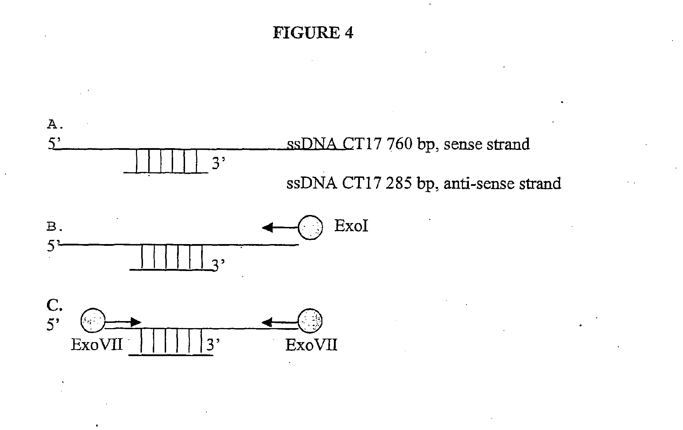

Two separate PCRs were performed, with one biotinylated primer and one unmodified primer, to create two different PCR products used as templates for ssDNA preparation (see table 14 and 15). After the ssDNA preparation, the two resulting ssDNAs of different sizes and polarities were hybridised. The resulting hybrid molecule was then treated with Exonuclease I and Exonuclease VII, respectively, and the digestion products run on an agarose gel to evaluate the results of the experiment (see figure 4).

**Table 14**

| | | | | |
|---|---|---|---|---|
| Primers used to make products and polarity of corresponding ssDNA. | | | | |

| *PCR product* | *Length* | *Primer 1* | *Primer 2* | *Polarity of ssDNA* |
|---|---|---|---|---|
| CT17 760bp | 760 bp | 127_5'VH biotine | 49_3'smuc159- | sense |
| CT17 285bp | 285bp | 149_5'CDR3VH-, biotine | 145_3'CDR1VL | Anti-sense |

**Table 15**

| | |
|---|---|
| Primer sequences | |

| *Primer name* | *Primer sequence* |
|---|---|
| 127 5'VH | 5'GAGGTGCAGCTGTTGGAGTCT |
| 49 3'smuc159-biotine | 5'Biotine-CAGCTTGGTTCCTCCGCCGAA |
| 149 5'CDR3VH-biotine | 5'Biotine-CGTGTATTACTGTGCGAGAGT |
| 145 3'CDR1VL | 5'TCCTGGGAGCTGCTGATACCA |

### Material and methods

**Table 16**

| | | | | | |
|---|---|---|---|---|---|
| *PCR amplification of CT17 760 bp.* | | | | | |
| (a) | | | | | |

| | µl | *20.5* | *C* | | *Final* |
|---|---|---|---|---|---|
| Aq | 63.75 | 1307 | | | |
| dNTP | 16 | 328 | 1.25 | mM | 0.1995 |
| 10x buffer | 10 | 205 | 10 | x | 0.99751 |
| Primer 1 | 5 | 102.5 | 20 | | 0.99751 |
| Primer 2 | 5 | 102.5 | 20 | µM | 0.99751 |
| DNA polymerase | 0.25 | 5.125 | 5 | U/µl | 0.012 |
| DNA | 0.25 | | 666.5 | ng/µl | 1.662 |
| Total volume | 100.25 | 2055 | | | |
| (b) | | | | | |
| | *DNA* | *Primer1* | | *Primer2* | |
| 1-20 | CT17/pFAB5C | 127 5'VH | | 49 3'smuc159-biotine | |
| 21 | Negative control | | | | |
| | | | | | |
| (c) | | | | | |
| PCR Program: | | | | | |
| | | 94 °C | | 30 sec | |
| 35x | | 55°C | | 30 sec | |
| | | 72°C | | 1 min | |

| | | | | | |
|---|---|---|---|---|---|
| DNA Polymerase Amplitaq (5U/µl), Applied Biosystems | | | | | |

PCR products are purified with JetQuick PCR purification system (Genomed). Total yield: 52.5 µg, conc: 132.6 ng/µl

**Table 17**

| | | | | | |
|---|---|---|---|---|---|
| *PCR amplification of CT17 285 bp.* | | | | | |
| (a) | | | | | |

| | *µl* | *20.5* | *C* | | *Final* |
|---|---|---|---|---|---|
| Aq | 63.5 | 1333.5 | | | |
| dNTP | 16 | 336 | 1.25 | mM | 0.2 |
| 10xbuffer | 10 | 210 | 10 | x | 1 |
| Primer 1 | 5 | 105 | 20 | | 1 |
| Primer 2 | 5 | 105 | 20 | µM | 1 |
| DNA | 0.25 | 5.25 | 5 | U/µl | 0.013 |
| polymerase | | | | | |
| DNA | 0.25 | | 666.5 | ng/µl | 1.666 |
| Total | 100 | 2100 | | | |
| volume | | | | | |
| | | | | | |
| (b) | | | | | |
| | *DNA* | *Primer1* | | *Prime2* | |
| 1-20 | CT17/pFAB5C | 145 3'CDR1VL | | 149 5'CDR3VH-biotine | |
| 21 | Negative control | | | | |
| (c) | | | | | |
| PCR Program: | | | | | |
| 1x | | 94 °C | | 2 min | |
| 35x | | 94 °C | | 1 min | |
| | | 55 °C | | 30 sec | |
| | | 72 °C | | 1 min | |
| 1x | | 72 °C | | 7 min | |

| | | | | | |
|---|---|---|---|---|---|
| DNA Polymerase Amplitaq (5U/µl), Applied Biosystems | | | | | |

PCR products are purified with JetQuick PCR purification system (Genomed).

**Table 18**

| | | | | | | | |
|---|---|---|---|---|---|---|---|
| *ssDNA preparation of CT17 760 bp and CT17 285 bp.* | | | | | | | |
| ssDNA preparation with MACS Streptavidin kit (Miltenyi Biotec, GTF) | | | | | | | |

| *Sample* | *dsDNA* | *Conc.* | *Length (ng*/*µL) (bp) x* | *Number of bp dsDNA* | *Amount dsDNA 0,066* (µg) (µl)* | *Volume dsDNA* | *Volume beads x 5** (µL)* |
|---|---|---|---|---|---|---|---|
| 1 | CT17 760bp | 132.6 | 760 | 50.16 | 52.1 | 394 | 150 |
| 2 | CT17 285bp | 52.8 | 285 | 18.81 | 21.1 | 400 | 450 |

| | | | | | | | |
|---|---|---|---|---|---|---|---|
| * 100µl beads bind up to Xµg DNA (X=number of bp x 0.066. **Beads are added in 5x surplus. | | | | | | | |

- The column was equilibrated with "equilibration buffer for nucleic acid applications" and 2x100 µl 1x B&W (5 mM Tris pH 7.5, 0.5 mM EDTA, 1 M NaCl) was subsequently allowed to run through the column.
- The dsDNA was mixed with beads and applied.
- The column was washed 4 times with 100 µl 1x B&W and the ssDNA was eluted with 150 µl 0.1 M NaOH (stored at -20°C, freshly thawed) after which 45 µl 0.33 M HCl and 15 µl 1M Tris-HCl pH 8.0 was added to the eluate to neutralize the ssDNA.

### Purification of ssDNA from gel with Recochip (TaKaRa)

65 µl ssDNA/well was run for 60 min at 100V on a 1% agarose /1x TAE gel. Recochip was inserted and run for 10 + 2 min at 100V with reversed polarity. DNA content in the recochip was verified by UV. The recochip was removed to a tube (provided) and the tube was centrifugated for 5 sec at 5000 rpm. After precipitated with 2.5 vol. 95% EtOH and 0.1 vol. 3 M NaAc pH 4.6 was CT17 760 and CT17 285 dissolved in 50µl and 35µl 10mM Tris pH 8.0. respectively.

**Table 19**

| | | | |
|---|---|---|---|
| *Test hybridisation in 10mM Tris or PCR-buffer. p64, 15-HeT* | | | |
| CT17 760bp: CT17 285bp are hybridised in a molar ratio of 1:2 | | | |

| *1.10mM Tris* | *Volume 2.* | *PCR buffer* | *Volume* |
|---|---|---|---|
| 75ng CT17 760bp | 1.46µl | 75ng CT17 760bp | 1.46µl |
| 75ng CT17 760bp | 1.46µl | 75ng CT17 760bp | 1.46µl |
| 58ng CT17 285bp | 0.8µl | 58ng CT17 285bp | 0.8µl |
| 10mM Tris, | 7.74µl | 10x PCR buffer | 1.0µl |
| pH 8.0 | | | |
| | | H₂O | 6.74µl |
| Total volume | 10.0µl | Total volume | 10.0µl |

Samples were hybridised in a PCR machine at 95°C for 5 minutes, followed by a heteroduplex step, consisting of 45 cycles of 1 minute each where the temperature is lowered by 1 °C for each cycle after which the samples were run on a 1.5% agarose gel.

**Table 20**

| | |
|---|---|
| *Hybridisation in PCR buffer.* | |
| CT17 760bp and CT17 285bp were hybridised in a molar ratio of 1:2. | |

| *1.* | *Volume* |
|---|---|
| 1µg CT17 760bp | 19.5µl |
| 0.769µg CT17 285bp | 10.6µl |
| 10xPCR buffer | 4.0µl |
| H2O | 5.9µl |
| Total volume | 40µl |

Final concentration of DNA in the sample was 44ng/µl.

The sample was hybridised in a PCR machine at 95°C for 5 minutes, followed by a heteroduplex step, consisting of 45 cycles of 1 minute each where the temperature is lowered by 1°C for each cycle. Precipitation was performed as described above and pellet dissolved in 40µl 10mM Tris, pH 8.

### Fragmentation of hybridised CT17 760bp-CT17 285bp with Exo I.

**Table 21**

| | *1* | *2* |
|---|---|---|
| DNA | Hybr. CT17 760bp/285bp | Hybr. CT17 760bp/285bp |
| Concentration (ng/µl) | 44 | 44 |
| Amount used (ng) | 748 | 60 |

**Table 22**

| | *1* | *2* |
|---|---|---|
| H₂O | 10.76 µl | 3.1 µl |
| 10x ExoI buffer (NEB) | 3.5 µl | 0.5 µl |
| ExoI (NEB) 10U/ul | 3.74 µl | - |
| ssDNA | 17.0 µl | 1.4 µl |
| | | |
| *Total* | 35 µl | 5 µl |

ExoI and hybridized DNA was added to the 37°C pre warmed water/buffer mixture. For sample 1, 17.5 µl was removed at 10min and 15min, respectively, and heat inactivated for 10 minutes at 96°C. For sample 2, the entire volume was removed and heat inactivated for 10 minutes at 96°C after 15min. The entire control reaction (60ng) and 2.8 µl (60ng) from 10 min and 15 min were run on an 1.2% agarose gel.

### Fragmentation of hybridised CT17 760bp-CT17 285bp with Exo VII

**Table 23**

| | *1* | *2* |
|---|---|---|
| DNA | Hybr. CT17 760bp/285bp | Hybr. CT17 760bp/285bp |
| Concentration (ng/µl) | 44 | 44 |
| Amount used (ng) | 748 | 60 |

### ExoVII

Concentration: 10 U/µl
Dilution: 2 U/µl
Produced by: USB
Lot number: 108705-005

**Table 24**

| | *1* | *2* |
|---|---|---|
| H₂O | 12.6 µl | 3.1 µl |
| 1 0x ExoI buffer* | 3.5 µl | 0.5 µl |
| ExoI | 1.87 µl | - |
| ssDNA | 17.0µl | 1.4 µl |
| | | |
| *Total* | 35 µl | 5 µl |

| | | |
|---|---|---|
| *10× Exo VII buffer: 500mM Tris HCl, pH 7.9, 500mM Potassium phosphate, pH 7.6, 83mM EDTA, 100xmM B-Mercaptoethanol | | |

- H₂O and buffer are pre-warmed for 10 min at 37°C.
- ExoVII is added.
- The hybridized DNA is added.
- For sample 1, 17.5 µl is taken out at 20 min and 30 min, respectively, and heat inactivated for 10 minutes at 96°C.
- For sample 2, the entire volume is taken out at 30 min and heat inactivated for 10 minutes at 96°C.
- The entire control reaction (60ng) and 2.8 µl (60ng) from 20 min and 30 min are run on a 1.2% agarose gel.

### Results

### Preparation of ssDNA

Two separate PCR reactions were made to produce the following PCR-products: CT17 760bp (3'biotinylated) and CT17 285bp (5'biotinylated). From these dsDNA templates ssDNAs was prepared (see Material and Methods, above).

### Test hybridisation of CT17 760bp and CT17 285bp

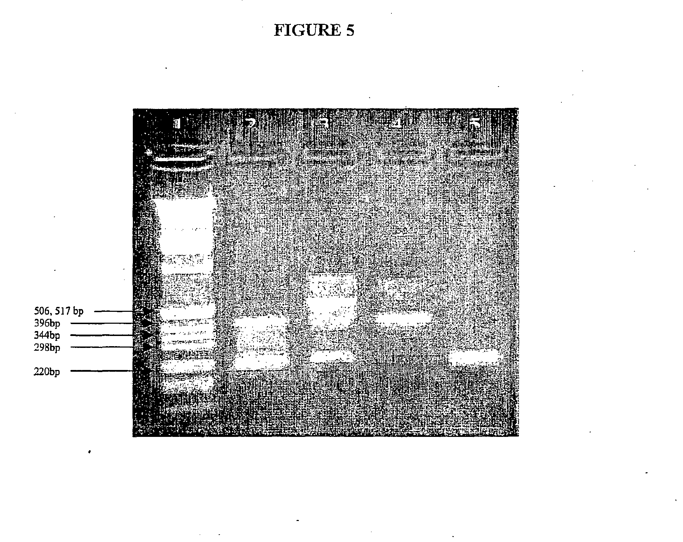

Two different hybridisation buffers were evaluated: 10mM Tris pH 8.0 and 1x PCR buffer (Applied Biosystems) (see Material and methods). The entire reactions were run on an agarose gel (see figure 5).

### Hybridisation of CT17 760bp and CT17 285bp and digestion with ExoI and ExoVII

CT17 760bp and CT17 285bp were hybridised in 1xPCR buffer with molar ration 1:5 and then digested with ExoI and ExoVII in two separate reactions (see Material and methods).

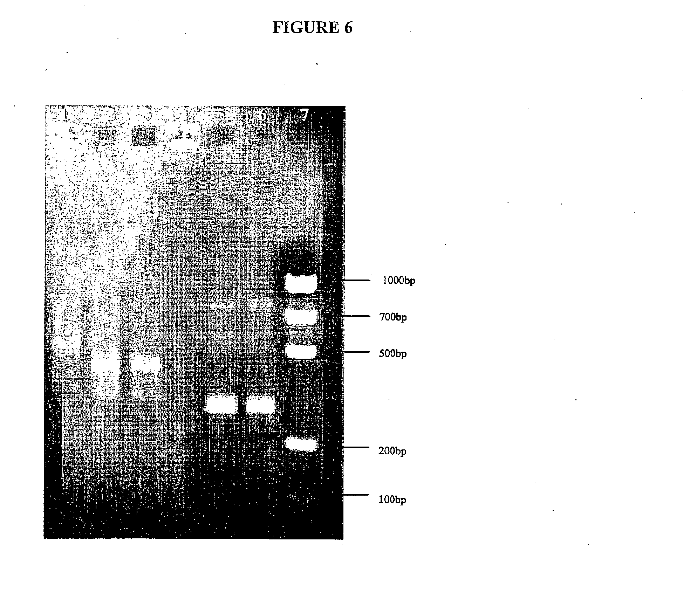

60 ng of each digestion product was run on an agarose gel (see figure 6).

### Discussion

The test hybridisation of the two fragments clearly shows that hybridisation occurs in the sample that has been hybridised in PCR buffer, where we see a band corresponding to the hybrid, with one region of dsDNA and an overhang of ssDNA on either side. This band is smaller than the expected size, 760bp, but this is probably due to altered migration properties conferred by the ssDNA overhangs. ssDNA migrates differently from dsDNA in an agarose gel, often migrating at about half the size of the corresponding dsDNA.

There has not been any hybridisation in the other sample, where we only see the bands corresponding to the two original ssDNAs. This demonstrates that the ionic strength of PCR buffer is adequate whereas 10mM Tris is not sufficient for hybridisation to take place. All further hybridisations have been done in PCR buffer.

The digestions with Exo I and ExoVII give the expected results: Exo I, which only digests from 3'→5', leaves a band where the ssDNA overhang on the 5'end is still present but is removed on the 3' end. This band is again smaller than the expected size (558bp) but the ssDNA overhang on the 5'end probably alters the mobility pattern in the gel. ExoVII, which digests from both 5'→3' and 3'→5,' removes all overhanging ssDNA and leaves only a dsDNA band of 285 bp.

These experiments clearly show that by hybridisation with a complementary ssDNA, selected areas in a nucleotide sequence can be protected from digestion with exonucleases.

## Claims

1. A method for generating a polynucleotide sequence or population of sequences from parent polynucleotide sequences, the method comprising the steps of
(a) providing a first population of polynucleotide molecules and a second population of polynucleotide molecules, the first and second populations together constituting plus and minus strands of a parent polynucleotide molecule;
(b) digesting the first and second populations of polynucleotide molecules with a nuclease to generate polynucleotide fragments;
(c) contacting said polynucleotide fragments generated from the plus strands with fragments generated from the minus strands (under conditions which permit annealing of fragments); and
(d) amplifying the fragments that anneal to each other to generate at least one polynucleotide molecule which differs in sequence from the parent polynucleotide molecule
wherein the degree of sequence variability in a selected region of the at least one polynucleotide molecule produced in step (d) is controlled by the addition of one or more oligonucleotides of predetermined variability, which oligonucleotides anneal to a sequence that lies between, but excludes, the 3'and 5' terminal nucleotides of the parent polynucleotide molecule.

2. A method according to Claim 1 wherein the parent polynucleotides encode one or more protein motifs.

3. A method according to Claim 1 or 2 wherein the first and second populations of polynucleotides are cDNA.

4. A method according to Claim 1, 2 or 3 wherein the first and second populations of polynucleotides are single-stranded.

5. A method according to any one of the preceding claims wherein the first population of polynucleotides consists of plus strands of parent polynucleotide sequences and second population of polynucleotides consists of minus strands of parent polynucleotide sequences.

6. A method according to any one of the preceding claims wherein the first and second populations of polynucleotides are digested separately in step (b).

7. A method according to any one of the preceding claims wherein the nuclease in step (b) is an exonuclease.

8. A method according to Claim 7 wherein the exonuclease is selected from the group consisting of BAL31, exonuclease I, exonuclease V, exonuclease VII, exonuclease T7 gene 6, bacteriophage lambda exonuclease and exonuclease Rec J_{f}.

9. A method according to any one of the preceding claims wherein the altered amino acid sequence of the at least one polynucleotide sequence produced in step (d) is associated with an altered property of the encoded polypeptide.

10. A method according to any one of Claims 4 to 9 wherein the oligonucleotides of predetermined variability are added prior to or in step (b) and wherein the nuclease is specific for single-stranded polynucleotides.

11. A method according to any one of Claims 1 to 9 wherein the oligonucleotides of predetermined variability are added after step (b) and prior to or in step (c).

12. A method according to any one of the preceding claims wherein the oligonucleotides of predetermined variability share at least 90% sequence identity with the internal sequence of a parent polynucleotide sequence, for example at least 95%, 96%, 97%, 98%, 99% or 100% sequence identity.

13. A method according to any one of the preceding claims wherein the oligonucleotides of predetermined variability share 100% sequence identity with the internal sequence of a parent polynucleotide sequence.

14. A method according to any one of the preceding claims wherein the oligonucleotides of predetermined variability are of a single nucleotide sequence.

15. A method according to any one of the preceding claims wherein the oligonucleotides of predetermined variability are of at least two different sequences.

16. A method according to Claim 15 wherein the oligonucleotides of predetermined variability are variants of the same internal sequence of a parent polynucleotide sequence.

17. A method according to Claim 15 wherein the oligonucleotides of predetermined variability share 100% sequence identity with, or are variants of, at least two different regions of the parent polynucleotides.

18. A method according to any one of the preceding claims wherein the oligonucleotides of predetermined variability are produced by error-prone PCR or using an oligonucleotide synthesiser.

19. A method according to any one of the preceding claims wherein the oligonucleotides of predetermined variability are between 10 and 500 nucleotides in length.

20. A method according to Claim 19 wherein the oligonucleotides of predetermined variability are between 50 and 200 nucleotides in length.

21. A method according to any one of Claims 1 to 20 wherein the parent polynucleotide sequences encode a ligand.

22. A method according to Claim 21 wherein the oligonucleotides of predetermined variability share sequence identity with, or are variants of, a region of the parent polynucleotide sequences encoding an amino acid sequence which interacts, directly or indirectly, with a biological molecule.

23. A method according to any one of Claims 1 to 20 wherein the parent polynucleotide sequences encode an antibody or antibody fragment.

24. A method according to Claim 23 wherein the oligonucleotides of predetermined variability share sequence identity with, or are variants of, a region of the parent polynucleotide sequences, encoding a framework polypeptide.

25. A method according to Claim 23 wherein the oligonucleotides of predetermined variability share sequence identity with, or are variants of, a region of the parent polynucleotide sequences encoding a CDR.

26. A method according to any one of Claims 1 to 20 wherein the parent polynucleotide sequences encode an enzyme or catalytically-active fragment thereof.

27. A method according to Claim 26 wherein the oligonucleotides of predetermined variability share sequence identity with, or are variants of, a region of the parent polynucleotide sequences encoding the active site, a modulatory site or a region involved in enzyme stability.

28. A method according to any one of Claims 1 to 20 wherein the parent polynucleotide sequences encode an antigen.

29. A method according to Claim 28 wherein the oligonucleotides of predetermined variability share sequence identity with, or are variants of, a region of the parent polynucleotide sequences encoding an epitope.

30. A method according to any one of the preceding claims wherein step (c) further comprises adding primer sequences that anneal to the 3'and/or 5'ends of at least one of the parent polynucleotides under annealing conditions.

31. A method according to any one of Claims 1 to 30 wherein, in step (b), at least one parameter of the reaction used for digestion of the first population of polynucleotide molecules is different from the equivalent parameter(s) used in the reaction for digestion of the second population of polynucleotide molecules.

32. A method according to Claim 31 wherein the reaction parameter is selected from nuclease type, nuclease concentration, reaction volume, duration of the digestion reaction, temperature of the reaction mixture, pH of the reaction mixture, length of parent polynucleotide sequences, the amount of parent polynucleotide molecules and the buffer composition of the reaction mixture.

33. A method according to any one of the preceding claims wherein the parent polynucleotide sequences have been subjected to mutagenesis.

34. A method according to any one of the preceding claims wherein one or both of the populations of fragments generated in step (b) are subjected to mutagenesis.

35. A method according to Claim 33 or 34 wherein the mutagenesis is error-prone PCR.

36. A method according to any one of the preceding claims wherein step (b) is carried out to generate populations of single-stranded fragments of varying lengths.

37. A method according to Claim 36 wherein step (b) is controlled to generate a population of single-stranded fragments having an average length of more than approximately 50 nucleotides.

38. A method according to any one of the preceding claims further comprising the step of expressing at least one polynucleotide sequence generated in step (d) to produce the encoded polypeptide.

39. A method according to Claim 28 further comprising the step of testing the encoded polypeptide for altered characteristics.

40. A method for making a polypeptide having altered properties, the method comprising the following steps:
| | |
|---|---|
| (a) | generating variant forms of a parent polynucleotide using a method according to any one of Claims 1 to 39; |
| (b) | expressing the variant polynucleotides produced in step (a) to produce variant polypeptides; |
| (c) | screening the variant polypeptides for altered properties; and |
| (d) | selecting a polypeptide having altered properties from the variant polypeptides. |

## Patentansprüche

1. Verfahren zum Generieren einer Polynukleotidsequenz oder einer Population von Sequenzen ausgehend von Ausgangspolynukleotidsequenzen, wobei das Verfahren die Schritte aufweist:
(a) Bereitstellen einer ersten Population von Polynukleotidmolekülen und einer zweiten Population von Polynukleotidmolekülen, wobei die erste Population und die zweite Population zusammen Plus- und Minusstränge eines Ausgangspolynukleotidmoleküls ausbilden;
(b) Aufschließen der ersten Population und der zweiten Population von Polynukleotidmolekülen mit einer Nuklease, um Polynukleotidfragmente zu generieren;
(c) Kontaktieren der Polynukleotidfragmente, die von den Plussträngen generiert wurden, mit Fragmenten, die ausgehend von den Minussträngen generiert wurden (unter Bedingungen, die das Hybridisieren von Fragmenten erlauben); und
(d) Verstärken der Fragmente, die miteinander hybridisieren, um mindestens ein Polynukleotidmolekül zu erzeugen, das sich in seiner Sequenz von dem Ausgangspolynukleotidmolekül unterscheidet,
wobei das Ausmaß der Sequenzvariabilität in einem ausgewählten Bereich des mindestens einen Polynukleotidmoleküls, das in Schritt (d) produziert wird, durch die Zugabe von einem oder mehreren Oligonukleotiden von vorbestimmter Variabilität gesteuert wird, welche Oligonukleotide mit einer Sequenz hybridisieren, die zwischen den 3'- und 5'-terminalen Nukleotiden des Ausgangspolynukleotidmoleküls liegt, aber diese ausschließt.

2. Verfahren nach Anspruch 1, wobei das Ausgangspolynukleotid ein oder mehrere Proteinmotive codiert.

3. Verfahren nach Anspruch 1 oder 2, wobei die erste Population und die zweite Population von Polynukleotiden cDNS sind.

4. Verfahren nach Anspruch 1, 2 oder 3, wobei die erste Population und zweite Population von Polynukleotiden einzelsträngig sind.

5. Verfahren nach einem der vorangehenden Ansprüche, wobei die erste Population von Polynukleotiden aus Plussträngen von Ausgangspolynukleotidsequenzen besteht und die zweite Population von Polynukleotiden aus Minussträngen von Ausgangspolynukleotidsequenzen besteht.

6. Verfahren nach einem der vorangehenden Ansprüche, wobei die erste Population und die zweite Population von Polynukleotiden in Schritt (b) voneinander getrennt aufgeschlossen werden.

7. Verfahren nach einem der vorangehenden Ansprüche, wobei die Nuklease in Schritt (b) eine Exonuklease ist.

8. Verfahren nach Anspruch 7, wobei die Exonuklease aus der Gruppe ausgewählt ist, die besteht aus BAL31, Exonuklease I, Exonuklease V, Exonuklease VII, Exonuklease T7-Gen6, Bakteriophage Lambda-Exonuklease und Exonuklease Rec J_{f}.

9. Verfahren nach einem der vorangehenden Ansprüche, wobei die geänderte Aminosäuresequenz der mindestens einen Polynukleotidsequenz, die in Schritt (d) produziert wird, mit einer geänderten Eigenschaft des codierten Polypeptids verbunden ist.

10. Verfahren nach einem der Ansprüche 4 bis 9, wobei die Oligonukleotide von vorbestimmter Variabilität vor oder in Schritt (b) zugegeben werden und wobei die Nuklease spezifisch für einzelsträngige Polynukleotide ist.

11. Verfahren nach einem der Ansprüche 1 bis 9, wobei die Oligonukleotide von vorbestimmter Variabilität nach Schritt (b) und vor oder in Schritt (c) zugegeben werden.

12. Verfahren nach einem der vorhergehenden Ansprüche, wobei die Oligonukleotide von vorbestimmter Variabilität mindestens 90 % Sequenzidentität mit der inneren Sequenz einer Ausgangspolynukleotidsequenz aufweisen, zum Beispiel mindestens 95 %, 96 %, 97 %, 98 %, 99 % oder 100 % Sequenzidentität.

13. Verfahren nach einem der vorhergehenden Ansprüche, wobei die Oligonukleotide von vorbestimmter Variabilität 100 % Sequenzidentität mit der inneren Sequenz einer Ausgangspolynukleotidsequenz aufweisen.

14. Verfahren nach einem der vorhergehenden Ansprüche, wobei die Oligonukleotide von vorbestimmter Variabilität von einer einzigen Nukleotidsequenz sind.

15. Verfahren nach einem der vorhergehenden Ansprüche, wobei die Oligonukleotide von vorbestimmter Variabilität von mindestens zwei unterschiedlichen Sequenzen sind.

16. Verfahren nach Anspruch 15, wobei die Oligonukleotide von vorbestimmter Variabilität Varianten derselben inneren Sequenz einer Ausgangspolynukleotidsequenz sind.

17. Verfahren nach Anspruch 15, wobei die Oligonukleotide von vorbestimmter Variabilität eine 100 % Sequenzidentität mit mindestens zwei unterschiedlichen Regionen des Ausgangspolynukleotids aufweisen oder Varianten davon sind.

18. Verfahren nach einem der vorhergehenden Ansprüche, wobei die Oligonukleotide von vorbestimmter Variabilität durch fehlerbehaftete (Error-prone) PKR hergestellt werden oder unter Verwendung eines Oligonukleotidsynthetisierers.

19. Verfahren nach einem der vorhergehenden Ansprüche, wobei die Oligonukleotide von vorbestimmter Variabilität zwischen 10 und 500 Nukleotide lang sind.

20. Verfahren nach Anspruch 19, wobei die Oligonukleotide von bestimmter Variabilität zwischen 50 und 200 Nukleotide lang sind.

21. Verfahren nach einem der Ansprüche 1 bis 20, wobei die Ausgangspolynukleotidsequenzen einen Liganden codieren.

22. Verfahren nach Anspruch 21, wobei die Oligonukleotide von vorbestimmter Variabilität Sequenzidentität mit einer Region der Ausgangspolynukleotidsequenz aufweisen, die eine Aminosäuresequenz codiert, welche direkt oder indirekt mit einem biologischen Molekül wechselwirkt, oder Varianten davon sind.

23. Verfahren nach einem Ansprüche 1 bis 20, wobei die Ausgangspolynukleotidsequenzen einen Antikörper oder ein Antikörperfragment codieren.

24. Verfahren nach Anspruch 23, wobei die Oligonukleotide von vorbestimmter Variabilität Sequenzidentität mit einer Region der Ausgangspolynukleotidsequenzen teilen, die ein Rahmenpolypeptid codiert, oder Varianten davon sind.

25. Verfahren nach Anspruch 23, wobei die Oligonukleotide von vorbestimmter Variabilität Sequenzidentität mit einer Region der Ausgangspolynukleotidsequenzen aufweisen, die einen CDR codiert, oder Varianten davon sind.

26. Verfahren nach einem der Ansprüche 1 bis 20, wobei die Ausgangspolynukleotidsequenzen ein Enzym oder ein katalytisch aktives Fragment davon codieren.

27. Verfahren nach Anspruch 26, wobei die Oligonukleotide von vorbestimmter Variabilität Sequenzidentität mit einer Region der Ausgangspolynukleotidsequenzen teilen, die den aktiven Platz, einen modulierenden Platz oder eine Region codiert, die in die Enzymstabilität verwickelt ist.

28. Verfahren nach einem der Ansprüche 1 bis 20, wobei die Ausgangspolynukleotidsequenzen ein Antigen codieren.

29. Verfahren nach Anspruch 28, wobei die Oligonukleotide von vorbestimmter Variabilität Sequenzidentität mit einer Region der Ausgangspolynukleotidsequenzen aufweisen, die ein Epitop codiert, oder Varianten davon sind.

30. Verfahren nach einem der vorangehenden Ansprüche, wobei Schritt (c) weiterhin das Hinzufügen von Primärsequenzen aufweist, die unter Hybridisierungsbedingungen mit den 3'-und/oder 5'-Enden von mindestens einem der Ausgangspolynukleotide hybridisieren.

31. Verfahren nach einem der Ansprüche 1 bis 30, wobei sich in Schritt (b) mindestens ein Parameter der Reaktion, die zum Aufschluss der ersten Population von Polynukleotidmolekülen verwendet wird, von dem äquivalenten Parameter unterscheidet, der bei der Reaktion zum Aufschluss der zweiten Population von Polynukleotidmolekülen verwendet wird.

32. Verfahren nach Anspruch 31, wobei der Reaktionsparameter ausgewählt ist aus Nuklease-Typ, Nuklease-Konzentration, Reaktionsvolumen, Dauer der Aufschlussreaktion, Temperatur der Reaktionsmischung, pH-Wert der Reaktionsmischung, Länge der Ausgangspolynukleotidsequenzen, der Menge an Ausgangspolynukleotidmolekülen und der Pufferzusammensetzung der Reaktionsmischung.

33. Verfahren nach einem der vorhergehenden Ansprüche, wobei die Ausgangspolynukleotidsequenzen Mutationserzeugung unterworfen worden sind.

34. Verfahren nach einem der vorhergehenden Ansprüche, wobei eine oder beide der Populationen von Fragmenten, die in Schritt (b) erzeugt werden, Mutationserzeugung unterworfen werden.

35. Verfahren nach Anspruch 33 oder 34, wobei die Mutationserzeugung fehlerbehaftete (Error-prone) PKR ist.

36. Verfahren nach einem der vorhergehenden Ansprüche, wobei Schritt (b) durchgeführt wird, um Populationen von einzelsträngigen Fragmenten von variierender Länge zu generieren.

37. Verfahren nach Anspruch 36, wobei Schritt (b) gesteuert wird, um eine Population von einzelsträngigen Fragmenten mit einer mittleren Länge von mehr als ungefähr 50 Nukleotiden zu generieren.

38. Verfahren nach einem der vorhergehenden Ansprüche, das weiterhin den Schritt des Exprimierens von mindestens einer Polynukleotidsequenz, die in Schritt (d) generiert wird, aufweist, um das codierte Polypeptid zu produzieren.

39. Verfahren nach Anspruch 28, das weiterhin den Schritt des Testens des codierten Polypeptids auf geänderte Eigenschaften aufweist

40. Verfahren zum Herstellen eines Polypeptids mit geänderten Eigenschaften, wobei das Verfahren die folgenden Schritte aufweist:
(a) Generieren von Variantenformen eines Ausgangspolynukleotids unter Verwendung eines Verfahrens nach einem der Ansprüche 1 bis 39;
(b) Exprimieren der Variantenpolynukleotide hergestellt in Schritt (a), um Variantepolypeptide zu produzieren;
(c) Screenen der Variantenpolypeptide nach geänderten Eigenschaften; und
(d) Auswählen eines Polypeptids mit geänderten Eigenschaften aus den Variantenpolypeptiden.

## Revendications

1. Procédé pour générer une séquence polynucléotidique ou une population de séquences provenant de séquences polynucléotidiques parentes, le procédé comprenant les étapes consistant à :
(a) fournir une première population de molécules polynucléotidiques et une seconde population de molécules polynucléotidiques, les première et seconde populations constituant ensemble des brins plus et moins d'une molécule polynucléotidique parente ;
(b) digérer les première et seconde populations de molécules polynucléotidiques avec une nucléase pour générer des fragments polynucléotidiques ;
(c) mettre en contact lesdits fragments polynucléotidiques générés à partir des brins plus avec des fragments générés à partir des brins moins (dans des conditions qui permettent l'hybridation des fragments) ; et
(d) amplifier les fragments qui s'hybrident les uns aux autres pour générer au moins une molécule polynucléotidique qui diffère en séquence de la molécule polynucléotidique parente
dans lequel le degré de variabilité de séquence dans une région sélectionnée de la au moins une molécule polynucléotidique produite dans l'étape (d) est régulé par l'addition d'un ou plusieurs oligonucléotides de variabilité prédéterminée, lesquels oligonucléotides s'hybrident à une séquence qui se situe entre les nucléotides 3' et 5' terminaux, exclus, de la molécule polynucléotidique parente.

2. Procédé selon la revendication 1, dans lequel les polynucléotides parents codent un ou plusieurs motifs de protéine.

3. Procédé selon la revendication 1 ou 2, dans lequel les première et seconde populations polynucléotidiques sont de l'ADNc.

4. Procédé selon la revendication 1, 2 ou 3, dans lequel les première et seconde populations polynucléotidiques sont simple brin.

5. Procédé selon l'une quelconque des revendications précédentes, dans lequel la première population de polynucléotides se compose de brins plus de séquences polynucléotidiques parentes et la seconde population de polynucléotides se compose de brins moins de séquences polynucléotidiques parentes.

6. Procédé selon l'une quelconque des revendications précédentes, dans lequel les première et seconde populations polynucléotidiques sont digérées séparément à l'étape (b).

7. Procédé selon l'une quelconque des revendications précédentes, dans lequel la nucléase à l'étape (b) est une exonucléase.

8. Procédé selon la revendication 7, dans lequel l'exonucléase est choisie dans le groupe constitué par BAL31, exonucléase I, exonucléase V, exonucléase VII, gène 6 d'exonucléase T7, exonucléase de bactériophage lambda et exonucléase Rec J*_{f}*.

9. Procédé selon l'une quelconque des revendications précédentes, dans lequel la séquence d'acides aminés modifiée de la au moins une séquence polynucléotidique produite à l'étape (d) est associée à une propriété modifiée du polypeptide codé.

10. Procédé selon l'une quelconque des revendications 4 à 9, dans lequel les oligonucléotides de variabilité prédéterminée sont ajoutés avant ou à l'étape (b) et dans lequel la nucléase est spécifique de polynucléotides simple brin.

11. Procédé selon l'une quelconque des revendications 1 à 9, dans lequel les oligonucléotides de variabilité prédéterminée sont ajoutés après l'étape (b) et avant ou à l'étape (c).

12. Procédé selon l'une quelconque des revendications précédentes, dans lequel les oligonucléotides de variabilité prédéterminée partagent au moins 90 % d'identité de séquence avec la séquence interne d'une séquence polynucléotidique parente, par exemple au moins 95 %, 96 %, 97 %, 98 %, 99 % ou 100 % d'identité de séquence.

13. Procédé selon l'une quelconque des revendications précédentes, dans lequel les oligonucléotides de variabilité prédéterminée partagent 100 % d'identité de séquence avec la séquence interne d'une séquence polynucléotidique parente.

14. Procédé selon l'une quelconque des revendications précédentes, dans lequel les oligonucléotides de variabilité prédéterminée proviennent d'une séquence nucléotidique unique.

15. Procédé selon l'une quelconque des revendications précédentes, dans lequel les oligonucléotides de variabilité prédéterminée proviennent d'au moins deux séquences différentes.

16. Procédé selon la revendication 15, dans lequel les oligonucléotides de variabilité prédéterminée sont des variants de la même séquence interne d'une séquence polynucléotidique parente.

17. Procédé selon la revendication 15, dans lequel les oligonucléotides de variabilité prédéterminée partagent 100 % d'identité de séquence avec, ou sont des variants d'au moins, deux régions différentes des polynucléotides parents.

18. Procédé selon l'une quelconque des revendications précédentes, dans lequel les oligonucléotides de variabilité prédéterminée sont produits par PCR sujette aux erreurs ou au moyen d'un synthétiseur d'oligonucléotide.

19. Procédé selon l'une quelconque des revendications précédentes, dans lequel les oligonucléotides de variabilité prédéterminée ont une longueur comprise entre 10 et 500 nucléotides.

20. Procédé selon la revendication 19, dans lequel les oligonucléotides de variabilité prédéterminée ont une longueur comprise entre 50 et 200 nucléotides.

21. Procédé selon l'une quelconque des revendications 1 à 20, dans lequel les séquences polynucléotidiques parentes codent un ligand.

22. Procédé selon la revendication 21, dans lequel les oligonucléotides de variabilité prédéterminée partagent une identité de séquence avec, ou sont des variants d'une, région des séquences polynucléotidiques parentes codant une séquence d'acides aminés qui interagit, directement ou indirectement, avec une molécule biologique.

23. Procédé selon l'une quelconque des revendications 1 à 20, dans lequel les séquences polynucléotidiques parentes codent un anticorps ou fragment d'anticorps.

24. Procédé selon la revendication 23, dans lequel les oligonucléotides de variabilité prédéterminée partagent une identité de séquence avec, ou sont des variants d'une, région des séquences polynucléotidiques parentes codant un polypeptide d'ossature.

25. Procédé selon la revendication 23, dans lequel les oligonucléotides de variabilité prédéterminée partagent une identité de séquence avec, ou sont des variants d'une, région des séquences polynucléotidiques parentes codant une CDR.

26. Procédé selon l'une quelconque des revendications 1 à 20, dans lequel les séquences polynucléotidiques parentes codent une enzyme ou un fragment catalytiquement actif de celle-ci.

27. Procédé selon la revendication 26, dans lequel les oligonucléotides de variabilité prédéterminée partagent une identité de séquence avec, ou sont des variants d'une, région des séquences polynucléotidiques parentes codant le site actif, un site modulatoire ou une région impliquée dans la stabilité enzymatique.

28. Procédé selon l'une quelconque des revendications 1 à 20, dans lequel les séquences polynucléotidiques parentes codent un antigène.

29. Procédé selon la revendication 28, dans lequel les oligonucléotides de variabilité prédéterminée partagent une identité de séquence avec, ou sont des variants d'une, région des séquences polynucléotidiques parentes codant un épitope.

30. Procédé selon l'une quelconque des revendications précédentes, dans lequel l'étape (c) comprend en outre l'ajout de séquences d'amorces qui s'hybrident aux extrémités 3' et/ou 5' d'au moins l'un des polynucléotides parents dans des conditions d'hybridation.

31. Procédé selon l'une quelconque des revendications 1 à 30, dans lequel, à l'étape (b), au moins un paramètre de la réaction utilisé pour la digestion de la première population de molécules polynucléotidiques est différent du ou des paramètres équivalents utilisés dans la réaction pour la digestion de la seconde population de molécules polynucléotidiques.

32. Procédé selon la revendication 31, dans lequel le paramètre de réaction est choisi parmi le type de nucléase, la concentration en nucléase, le volume réactionnel, la durée de la réaction de digestion, la température du mélange réactionnel, le pH du mélange réactionnel, la longueur des séquences polynucléotidiques parentes, la quantité de molécules polynucléotidiques parentes et la composition tampon du mélange réactionnel.

33. Procédé selon l'une quelconque des revendications précédentes, dans lequel les séquences polynucléotidiques parentes ont été soumises à une mutagenèse.

34. Procédé selon l'une quelconque des revendications précédentes, dans lequel l'une des populations de fragments, ou les deux, générées à l'étape (b) sont soumises à une mutagenèse.

35. Procédé selon la revendication 33 ou 34, dans lequel la mutagenèse est une PCR sujette aux erreurs.

36. Procédé selon l'une quelconque des revendications précédentes, dans lequel l'étape (b) est réalisée pour générer des populations de fragments simple brin de longueurs variables.

37. Procédé selon la revendication 36, dans lequel l'étape (b) est régulée pour générer une population de fragments simple brin ayant une longueur moyenne de plus d'approximativement 50 nucléotides.

38. Procédé selon l'une quelconque des revendications précédentes, comprenant en outre l'étape consistant à exprimer au moins une séquence polynucléotidique générée à l'étape (d) pour produire le polypeptide codé.

39. Procédé selon la revendication 28, comprenant en outre l'étape consistant à évaluer les caractéristiques modifiées du polypeptide codé.

40. Procédé pour préparer un polypeptide ayant des propriétés modifiées, le procédé comprenant les étapes suivantes consistant à :
(a) générer des formes variantes d'un polynucléotide parent au moyen d'un procédé selon l'une quelconque des revendications 1 à 39 ;
(b) exprimer les polynucléotides variants produits à l'étape (a) pour produire des polypeptides variants ;
(c) cribler les propriétés modifiées des polypeptides variants ; et
(d) sélectionner un polypeptide ayant des propriétés modifiées à partir des polypeptides variants.


### SEQUENCE LISTING

<110> Alligator Bioscience AB
<120> A method for in vitro molecular evolution of protein function
<130> ALLBA/P36652PC
<150> 0523582.5
   <151> 19 November 2005
<160> 21
<170> seqwin99
<210> 1
   <211> 44
   <212> DNA
   <213> Artificial Sequence
<220>
   <223> 1736 sfiI forward primer
<400> 1
   attactcgcg gcccagccgg ccatggccca caggtcaagc tcga 44
<210> 2
   <211> 35
   <212> DNA
   <213> Artificial Sequence
<220>
   <223> 1735 NotI reversed primer
<400> 2
   ttagagcctg cggccgcctt gtcatcgtcg tcctt 35
<210> 3
   <211> 44
   <212> DNA
   <213> Artificial sequence
<220>
   <223> 1644 sfiI forward primer
<400> 3
   attactcgcg gcccagccgg ccatggccca caggtcaagc tcga 44
<210> 4
   <211> 35
   <212> DNA
   <213> Artificial Sequence
<220>
   <223> 1635 NotI reversed primer
<400> 4
   ttagagcctg cggccgcctt gtcatcgtcg tcctt 35
<210> 5
   <211> 45
   <212> DNA
   <213> Artificial Sequence
<220>
   <223> PCR primer 137
<400> 5
   cgcgaattgg cccagccggc catggccgag gtgcagctgt tggag 45
<210> 6
   <211> 30
   <212> DNA
   <213> Artificial Sequence
<220>
   <223> PCR primer 138
<400> 6
   agatggggga ctagtgctgc tcacggtgac. 30
<210> 7
   <211> 20
   <212> DNA
   <213> Artificial Sequence
<220>
   <223> PCR primer 350
<400> 7
   cctggagcct ggcggaccca 20
<210> 8
   <211> 20
   <212> DNA
   <213> Artificial Sequence
<220>
   <223> PCR primer 351
<400> 8
   gctgggtccg ccaggctcca 20
<210> 9
   <211> 20
   <212> DNA
   <213> Artificial sequence
<220>
   <223> PCR primer 354
<220>
   <221> MISC_FEATURE
   <222> 1..1
   <223> Biotinylated
<400> 9
   gactctcctg tgcagcctct 20
<210> 10
   <211> 20
   <212> DNA
   <213> Artificial sequence
<220>
   <223> PCR primer 357
<220>
   <221> MISC_FEATURE
   <222> 1..1
   <223> Biotinylated
<400> 10
   ttgtctctgg agatggtgaa 20
<210> 11
   <211> 20
   <212> DNA
   <213> Artificial Sequence
<220>
   <223> PCR primer 226
<400> 11
   ctcactatag ggcgaattgg 20
<210> 12
   <211> 28
   <212> DNA
   <213> Artificial Sequence
<220>
   <223> PCR primer 415
<400> 12
   ttcagatctc gaggtgcagc tgttggag 28
<210> 13
   <211> 19
   <212> DNA
   <213> Artificial sequence
<220>
   <223> PCR primer 224
<400> 13
   cctattgcct acggcagcc 19
<210> 14
   <211> 19
   <212> DNA
   <213> Artificial Sequence
<220>
   <223> PCR primer 332
<220>
   <221> MISC_FEATURE
   <222> 1..1
   <223> Biotinylated
<400> 14
   cctattgcct acggcagcc 19
<210> 15
   <211> 20
   <212> DNA
   <213> Artificial Sequence
<220>
   <223> PCR primer 333
<220>
   <221> MISC_FEATURE
   <222> 1..1
   <223> Biotinylated
<400> 15
   ctcactatag ggcgaattgg 20
<210> 16
   <211> 20
   <212> DNA
   <213> Artificial Sequence
<220>
   <223> PCR primer 349
<400> 16
   gactctcctg tgcagcctct 20
<210> 17
   <211> 20
   <212> DNA
   <213> Artificial Sequence
<220>
   <223> PCR primer 352
<400> 17
   ttgtctctgg agatggtgaa 20
<210> 18
   <211> 20
   <212> DNA
   <213> Artificial Sequence
<220>
   <223> PCR primer 355
<220>
   <221> MISC_FEATURE
   <222> 1..1
   <223> Biotinylated
<400> 18
   cctggagcct ggcggaccca 20
<210> 19
   <211> 20
   <212> DNA
   <213> Artificial Sequence
<220>
   <223> PCR primer 356
<220>
   <221> MISC_FEATURE
   <222> 1..1
   <223> Biotinylated
<400> 19
   gctgggtccg ccaggctcca 20
<210> 20
   <211> 19
   <212> DNA
   <213> Artificial Sequence
<220>
   <223> PCR primer 384
<400> 20
   cactgccgtg tattactgt 19
<210> 21
   <211> 18
   <212> DNA
   <213> Artificial Sequence
<220>
   <223> PCR primer 386
<220>
   <221> MISC_FEATURE
   <222> 1..1
   <223> Biotinylated
<400> 21
   cagtgtacct tggcccca 18